# FASCICULE DE BREVET EUROPEEN

(11) **EP 2 800 806 B1**
(45) Date de publication et mention de la délivrance du brevet: **25.05.2016**
(21) Numéro de dépôt: 13702246.3
(22) Date de dépôt: 04.01.2013
(51) Int. Cl.: C12M 1/00

(54) **CONTENEUR POUR CONTENU BIOPHARMACEUTIQUE**
BEHÄLTER FÜR BIOPHARMAZEUTISCHE INHALTE
CONTAINER FOR BIOPHARMACEUTICAL CONTENT

(30) Priorité: 05.01.2012 FR 1250124
(43) Date de publication de la demande: 12.11.2014
(73) Titulaire: SARTORIUS STEDIM FMT SAS, 13400 Aubagne (FR)
(72) Inventeur: CHAUSSIN, Sébastien, 13400 Aubagne (FR)
(74) Mandataire: Derambure Conseil
(86) Numéro de dépôt international: PCT/FR2013/050014
(87) Numéro de publication internationale: WO 2013/102738

(56) Documents cités:
- WO-A1-2010/106282
- US-B1- 6 367 745
- US-B2- 8 028 962

## Description

L'invention est relative au domaine technique des conteneurs pour la réception de contenu(s) biopharmaceutique(s).

Elle vise plus particulièrement le domaine des conteneurs destinés à recevoir un contenu biopharmaceutique et comprenant au moins un conduit de transfert pour le transport de produits de transfert ainsi qu'un ou plusieurs élément(s) de guidage supporté(s) par le conteneur et destiné(s) à maintenir le conduit de transfert en position vis-à-vis de la paroi périphérique du conteneur.

Dans ce domaine technique, il est connu de l'art antérieur différentes solutions visant à répondre aux problématiques du maintien des conduits de transfert en position vis-à-vis du conteneur.

Notamment, un conteneur selon le préambule de la revendication 1 est connu de l'homme du métier par l'exemple qu'en donne le document WO-A1-2010106282. Ce document décrit, en effet, un récipient-mélangeur comprenant un conteneur flexible destiné à recevoir un contenu biopharmaceutique, des moyens de mélange du contenu biopharmaceutique et des moyens d'aération formés pour partie par un élément tubulaire s'étendant jusqu'à des moyens de distribution d'air situés à l'intérieur du conteneur. L'élément tubulaire est, en outre, maintenu en position dans le conteneur au moyen d'éléments de guidage fixés par collage, soudage, etc. contre la paroi périphérique. Ces éléments de guidage sont susceptibles d'être réalisés à partir de bandes d'adhésif, de brides, de cavaliers disposés de place en place le long de l'élément tubulaire d'aération.

Toutefois, une telle réalisation présente plusieurs inconvénients. D'abord, la mise en place des conduits de transfert à l'intérieur des éléments de guidage - une fois que ces derniers sont en position contre la paroi périphérique - n'est pas aisée car les éléments de guidage sont souvent difficilement accessibles à l'intérieur du conteneur. Il peut ainsi s'avérer nécessaire d'entrer dans le conteneur pour associer les conduits de transfert aux éléments de guidage, ce qui n'est pas ergonomique et engendre des problèmes de contamination. De plus, la mise en place des conduits de transfert dans les éléments de guidage peut s'avérer délicate et implique des contraintes fortes et répétées sur la paroi périphérique du conteneur, pouvant provoquer des défauts d'aspect, voir des défauts structurels nuisant à l'étanchéité de ce dernier. Compte-tenu de ces problématiques, il peut arriver que les conduits de transfert soient mal positionnés, causant ainsi des difficultés lors de l'utilisation des conteneurs. Par ailleurs, les problèmes rencontrées par les opérateurs lors de la mise en place des conduits de transfert dans les éléments de guidage nuit à leur productivité dans le cadre de la fabrication des conteneurs.

Le document WO-A1-2009116002 se rapporte à un conteneur similaire à celui présenté précédemment et comporte, par conséquent, les mêmes limites et inconvénients.

Il est aussi connu de l'art antérieur la gamme de produits CULTIBAG STR® commercialisés par la demanderesse et qui constitue une autre forme de conteneur pour la réception de contenus biopharmaceutiques. Ce conteneur CULTIBAG STR® comprend une paroi périphérique composée de films plastiques soudés les uns aux autres de manière à définir une chambre interne de réception pour le contenu biopharmaceutique. Il comporte également des conduits de transfert pour le transport de produits de transfert ainsi que des boucles de guidage destinées à assurer le guidage de ces conduits de transfert à l'intérieur du conteneur. Plus particulièrement, les boucles de guidage sont formées par des bandes en matériau plastique, dont les deux extrémités sont prises en sandwich au niveau des zones de soudure des films plastiques formant la paroi périphérique du conteneur. Les conduits de transfert peuvent ainsi être passés dans les boucles de guidage pour garantir leur position à proximité des zones de soudures de la paroi périphérique.

Une telle réalisation présente cependant des inconvénients. D'abord, la mise en place des conduits de transfert à l'intérieur des boucles de guidage est délicate, et ce d'autant plus que la position des boucles de guidage est déterminée par celle des zones de soudure de la paroi périphérique du conteneur. Dès lors, l'accès à ces boucles de guidage peut, en pratique, s'avérer difficile. Comme précédemment, la mise en place des conduits de transfert dans les boucles de guidage engendre également des contraintes fortes et répétées sur les zones de soudure du conteneur ce qui peut provoquer des défauts d'étanchéité. En outre, une telle réalisation impose d'utiliser des boucles de guidage suffisamment large pour permettre le passage des conduits de transfert. Il en résulte que le guidage de ces conduits de transfert dans la chambre interne de réception est imprécis et peut générer, selon les cas, une tension excessive sur les boucles de guidage ou bien au contraire des zones lâches. Il est donc impossible de garantir la position exacte des conduits de transferts ainsi que les zones d'introduction et de retrait du ou des produit(s) de transfert. En outre, la fixation des boucles de guidage entre les films plastiques de la paroi périphérique des conteneurs impose d'utiliser des paramètres de soudure spécifiques et complexes, ce qui peut engendrer des difficultés dans le cadre du processus de production et oblige à une vigilance particulière quant aux défauts de fabrication.

Il est, par ailleurs, connu du document US-A-5,362,642, un conteneur destiné à la réception de contenus biopharmaceutiques, comprenant une paroi périphérique ainsi que des conduits de transfert pour l'introduction et/ou le retrait de produits de transfert. Cependant, dans le cas d'espèce, les conduits de transfert ne sont pas maintenus contre la paroi du conteneur et leur position n'est donc contraint que par la force de gravité qui les pousse à s'étendre partiellement à la verticale.

Une telle réalisation n'assure donc pas un positionnement précis des conduits de transfert qui ne suivent pas une trajectoire prédéterminée. Il est donc impossible d'introduire ou d'extraire les produit(s) de transfert à des emplacements prédéterminés.

Dans un domaine technique totalement différent qu'est celui du support et du guidage de fils électriques, il est connu de l'art antérieur le document US-B2-8,028,962. Ce document décrit un outil de fixation destiné à soutenir solidement des fils électriques sur un élément de support. Plus particulièrement, l'outil de fixation comprend une boucle reliée à une tige de fixation pouvant être fixée dans un trou de l'élément de support. L'outil de fixation comporte en outre, une lanière réglable destinées, d'abord, à être introduite dans la boucle et, ensuite, à être enserrée autour des fils électriques pour les maintenir en position.

Toujours dans ce domaine technique éloigné du maintien de fils électriques, on connait également US 6,367,745, qui décrit un outil de fixation comprenant une boucle reliée à une base de fixation. La base de fixation est montée en force par l'arrière dans un panneau mince. L'outil de fixation comporte en outre, une lanière destinée, d'abord, à être introduite dans la boucle et, ensuite, à être enserrée autour des fils électriques pour les maintenir en position.

De telles réalisations, outre qu'elles appartiennent à un domaine technique éloigné de celui de l'invention, ne permettent donc pas de mettre en place simplement les fils électriques lorsque l'outil de fixation doit être placé dans une position difficilement accessible.

Dans ce contexte, la présente invention a pour but de proposer un conteneur pour la réception de produits biopharmaceutiques exempt de l'une au moins des limitations précédemment évoquées.

À cette fin, l'invention vise selon un premier aspect un conteneur pour la réception d'un contenu biopharmaceutique sous forme liquide, fluide ou bien gazeuse, comprenant: une paroi périphérique refermée sur elle-même et définissant une chambre interne de réception capable de recevoir le contenu biopharmaceutique ; au moins un conduit de transfert présentant une portion extrême proximale ouverte sur la chambre interne de réception et une portion extrême distale ouverte sur l'extérieur du conteneur de manière à autoriser le transport d'un produit de transfert entre ladite chambre interne de réception et l'extérieur dudit conteneur ; au moins un élément de guidage apte à être supporté par la paroi périphérique et à maintenir le au moins un conduit de transfert en position à proximité de cette paroi périphérique de manière à imposer une trajectoire prédéterminée entre la portion extrême proximale et la portion extrême distale. Le au moins un élément de guidage comporte, d'une part, un organe de préhension capable d'être solidarisé structurellement audit au moins un conduit de transfert et, d'autre part, un organe de fixation apte à se fixer sur n'importe quelle zone de liaison prédéterminée de la paroi périphérique sans affecter son étanchéité. Par ailleurs, l'invention se caractérise en ce que l'organe de préhension et l'organe de fixation sont structurellement distincts et indépendants l'un de l'autre et en ce que l'élément de guidage présente des moyens d'assemblage réciproques permettant d'assurer l'assemblage structurel ou fonctionnel de l'organe de préhension, lorsque ce dernier est solidaire de l'au moins un conduit de transfert, avec l'organe de fixation, lorsque ce dernier est fixé sur la zone de liaison, pour garantir le positionnement dudit organe de préhension à proximité dudit organe de fixation et de la zone de liaison de la paroi périphérique correspondante.

Grâce à cet agencement, il est possible de faciliter la mise en place des conduits de transfert dans la chambre interne de réception puisque l'opérateur peut réaliser, dans un premier temps, la préhension des conduit(s) de transfert et la fixation de l'organe de fixation contre la paroi périphérique et, dans un second temps, l'assemblage simplifié de l'organe de préhension et de l'organe de fixation. Ainsi, les risques de contamination de l'espace interne de réception et les défauts d'aspects ou structurels susceptibles d'être générés lors de cette opération d'assemblage sont largement réduits. Par ailleurs, le fait de pouvoir utiliser l'organe de préhension préalablement à son association avec l'organe de fixation permet de réaliser une préhension efficace des conduits de transfert, de limiter les risques de positionnement inadaptés et donc de garantir un degré de précision plus élevé. De la même manière, pouvoir solidariser l'organe de fixation à la paroi périphérique du conteneur préalablement à son association avec l'organe de préhension et les conduits de transfert simplifie, facilite, et accélère cette étape du procédé de fabrication.

Selon une réalisation, l'organe de fixation présente une base de fixation dotée d'une tenue intrinsèque suffisante pour éviter toute déformation structurelle, en l'absence de sollicitation extérieure. Contrairement aux lanières flexibles dont le soudage ou le collage sur la paroi périphérique du conteneur est malaisé, l'utilisation d'une base de fixation dotée d'une tenue intrinsèque permet d'envisager la solidarisation de cette base de fixation contre la paroi périphérique dans des conditions privilégiées.

Dans ce cas, selon une première réalisation, la base de fixation présente une face plate apte à être fixée sur l'une des zones de liaison de la paroi périphérique par un procédé de fixation irréversible tel que le soudage, le collage, ou analogue.

Dans ce cas, selon une deuxième réalisation, la base de fixation et une contre-pièce de fixation sont aptes à être positionnées de part et d'autre de la paroi périphérique de manière à enserrer l'une des zones de liaison de la paroi périphérique et à assurer le maintien en position de l'organe de fixation sur ladite zone de liaison.

Plus particulièrement, selon une première variante de réalisation, la base de fixation et la contre-pièce de fixation présentent des profils géométriques complémentaires pouvant d'être assemblés mécaniquement l'un à l'autre, de manière réversible ou irréversible, sans traverser la paroi périphérique ni altérer la continuité structurelle de cette dernière. Une telle réalisation permet d'éviter la réalisation d'une opération de soudage ou de collage tout en assurant un maintien en position précis et modulable de l'organe de fixation.

Å l'inverse, selon une seconde variante de réalisation, la paroi périphérique présente au moins une ouverture de fixation, l'une ou l'autre de la base de fixation et de la contre-pièce de fixation étant apte à être fixée autour de l'ouverture de fixation de manière à assurer l'étanchéité de l'espace interne de réception définit par la paroi périphérique, l'une ou l'autre de ladite base de fixation et de ladite contre-pièce de fixation étant apte à traverser l'ouverture de fixation, la base de fixation et la contre-pièce de fixation présentant des profils géométriques complémentaires pouvant être assemblés mécaniquement l'un à l'autre, de manière réversible ou irréversible. Cette deuxième variante est également avantageuse car elle permet d'obtenir un assemblage efficace de la base de fixation avec la contre-pièce de fixation dans la mesure où ces deux pièces peuvent entrer en contact structurel direct l'une avec l'autre.

Selon une réalisation, l'organe de préhension est formé par des moyens d'encerclement aptes à enserrer une portion d'accouplement du au moins un conduit de transfert. Cela permet notamment d'obtenir un organe de préhension modulable, précis et pouvant être utilisé pour enserrer un ou plusieurs conduit(s) de transfert tout en limitant les risques d'écrasement.

Dans ce cas, selon une réalisation, les moyens d'encerclement présentent des moyens de serrage aptes à enserrer l'au moins un conduit de transfert en s'ajustant à ses dimensions ainsi que des moyens de blocage en position serrée aptes à bloquer les moyens de serrage lorsque les moyens d'encerclement sont ajustés aux dimensions de l'au moins un conduit de transfert sans l'écraser ou le plier sous l'effet de la contrainte de serrage. Une telle réalisation est particulièrement efficace pour obtenir une préhension précise et qui s'adapte sans difficulté aux formes du ou des conduit(s) de préhension.

Dans ce cas, selon une réalisation alternative ou complémentaire, les moyens d'encerclement présentent une élasticité leur permettant d'enserrer le au moins un conduit de transfert sans l'écraser ou le plier sous l'effet de la contrainte de serrage. Il est ainsi possible d'augmenter la contrainte de serrage en limitant les risques d'écrasement

Selon un premier mode de réalisation, les moyens d'assemblage réciproques sont formés, pour partie, sur l'organe de préhension et, pour partie complémentaire, sur l'organe de fixation de sorte à assurer une liaison mécanique directe entre ledit organe de préhension et ledit organe de fixation. Le nombre de pièces utilisées est alors restreint et la fabrication comme l'assemble de l'élément de guidage est simplifié.

Selon un deuxième mode de réalisation alternatif, les moyens d'assemblage réciproques comprennent un organe intermédiaire de liaison distinct dudit organe de préhension et dudit organe de fixation. Il est ainsi possible d'utiliser un organe de préhension très simple - comme, par exemple, une boucle de serrage - tout en utilisant un organe intermédiaire de liaison capable de coopérer de manière plus complexe avec l'organe de fixation afin de simplifier leur assemblage.

A cet égard, selon une réalisation l'organe intermédiaire de liaison présente des moyens de solidarisation à l'organe de fixation aptes à assurer, entre l'organe intermédiaire de liaison et l'organe de fixation, une liaison mécanique choisit parmi une liaison complète qui a l'avantage d'être simple à réaliser, une liaison rotule ou une liaison rotule à doigt qui permet d'obtenir une certaine souplesse dans le position et l'orientation de l'organe de préhension et du ou de(s) conduits de transfert, et une liaison pivot qui favorise le positionnement de l'organe de préhension et du ou de(s) conduits de transfert, notamment si l'orientation de ces derniers génère des difficultés de mise en place.

Dans l'hypothèse d'utilisation de l'organe intermédiaire de liaison et selon une variante, l'un de l'organe intermédiaire de liaison et de l'organe de fixation forme un élément mâle et l'autre un élément femelle, l'un des éléments mâle et femelle présentant une souplesse propre à autoriser la liaison mécanique de l'organe de fixation à l'organe intermédiaire de liaison par encliquetage en force dudit élément mâle dans l'élément femelle. Il est ainsi particulièrement simple et rapide d'assurer l'assemblage fonctionnel de l'organe de préhension avec l'organe de fixation.

Toujours dans cette hypothèse d'utilisation de l'organe intermédiaire de liaison et selon une autre variante, l'organe intermédiaire présente des moyens de solidarisation à l'organe de préhension.

Dans ce cas, les moyens de solidarisation à l'organe de préhension sont formés par au moins une boucle solidaire de l'organe intermédiaire et dans laquelle peut passer l'organe de préhension. Une telle réalisation est particulièrement simple à réaliser à mettre en oeuvre, notamment en ce qui concerne la préhension du ou des conduit(s) de transfert.

Dans l'hypothèse d'utilisation de l'organe intermédiaire de liaison et selon une autre variante, ce dernier présente une portion formant berceau de réception apte à épouser la forme de la portion d'accouplement de l'au moins un conduit de transfert lorsque ce dernier est enserré par l'organe de préhension. Il est ainsi possible d'augmenter les contraintes de serrage du ou des conduit(s) de transfert pour garantir sa position tout en limitant les risques d'écrasement dudit ou desdits conduit(s) de transfert.

Selon une réalisation, le au moins un élément de guidage est fixé sur la face interne ou la face externe de la paroi périphérique. Il peut, en effet, également s'avérer intéressant d'assurer le guidage d'un ou plusieurs conduit(s) de transfert sur la face externe de la paroi périphérique du conteneur.

Selon une réalisation, le conteneur comprend n conduits de transfert, n étant un entier supérieur à 1, et au moins une entretoise de cloisonnement présentant m sièges de réception, m étant un entier supérieur à 1, aptes à recevoir les n conduits de transfert et à empêcher le contact desdits n conduits de transfert les uns avec les autres. En évitant ainsi le contact des conduits de transfert les uns par rapport aux autres, il est possible de réduire les risques d'écrasement de ces derniers.

Dans ce cas, selon une réalisation, les m sièges de réception sont régulièrement répartis sur le pourtour de l'entretoise de sorte à équilibrer les contraintes de serrage imposées sur les n conduits de transfert lorsque ces derniers sont en position dans les sièges de réception. De la même manière, une telle disposition permet de limiter la concentration des contraintes de serrage sur une portion d'accouplement de l'un des conduits de transfert et donc de réduire les risques d'écrasement de ces conduits de transfert.

Selon une réalisation, un premier élément de guidage est supporté par une première zone de liaison de la paroi périphérique et enserre la portion extrême proximale d'un premier conduit de transfert de sorte à la guider jusqu'à un premier emplacement correspondant à la zone d'introduction ou de retrait d'un premier produit de transfert. Il est, de cette manière, possible de retirer ou d'introduire le produit de transfert à un emplacement précis du conteneur et, donc, d'améliorer sensiblement les paramètres de la réaction en cours dans l'espace interne de réception du conteneur.

Dans ce cas, selon une réalisation, le conteneur comprend un deuxième élément de guidage distinct du premier élément de guidage supporté par une deuxième zone de liaison de la paroi périphérique et enserre la portion extrême proximale d'un deuxième conduit de transfert de manière à la guider jusqu'à un deuxième emplacement distinct du premier emplacement et correspondant à la zone d'introduction ou de retrait d'un deuxième produit de transfert. Il est alors possible de générer des réactions plus efficaces, tenant compte de l'emplacement d'introduction ou de retrait de plusieurs produits de transfert.

L'invention concerne, selon un deuxième aspect, un procédé de réalisation d'un conteneur pour la réception d'un contenu biopharmaceutique sous forme liquide, fluide ou bien gazeuse, selon le premier aspect de l'invention, caractérisé en ce qu'il comprend une succession d'étapes consistant à : disposer d'une paroi périphérique destinée à être refermée sur elle-même pour définir une chambre interne de réception capable de recevoir le contenu biopharmaceutique ; solidariser structurellement au moins un conduit de transfert présentant une portion extrême proximale et une portion extrême distale à un organe de préhension ; fixer un organe de fixation structurellement distincts et indépendants de l'organe de préhension sur n'importe quelle zone de liaison prédéterminée de la paroi périphérique sans affecter son étanchéité ; puis, assembler de façon structurelle ou fonctionnelle ledit organe de préhension avec ledit organe de fixation pour former un élément de guidage permettant de garantir le positionnement dudit organe de préhension et de l'au moins un conduit de transfert qui lui est solidaire à proximité dudit organe de fixation et de la zone de liaison de la paroi périphérique correspondante.

Dans ce cas, selon une première variante de réalisation, l'organe de fixation présente une base de fixation dotée d'une face plate qui est fixée sur l'une des zones de liaison de la paroi périphérique par un procédé de fixation irréversible tel que le soudage, le collage, ou analogue.

Dans ce cas, selon une deuxième variante de réalisation, l'organe de fixation présente une base de fixation et une contre-pièce de fixation qui sont positionnées de part et d'autre de la paroi périphérique et sont assemblées mécaniquement l'un à l'autre, de manière réversible ou irréversible, sans traverser la paroi périphérique ni altérer la continuité structurelle de cette dernière de sorte à enserrer l'une des zones de liaison de la paroi périphérique et assurer le maintien en position de l'organe de fixation sur ladite zone de liaison.

Alors, selon une réalisation, la paroi périphérique présente au moins une ouverture de fixation et l'organe de fixation présente une base de fixation et une contre-pièce de fixation, l'une ou l'autre de la base de fixation et de la contre-pièce de fixation étant fixée autour de l'ouverture de fixation de manière à assurer l'étanchéité de l'espace interne de réception définit par la paroi périphérique, l'une ou l'autre de ladite base de fixation et de ladite contre-pièce de fixation étant positionnée de manière à traverser l'ouverture de fixation, la base de fixation et la contre-pièce de fixation étant assemblées mécaniquement l'un à l'autre, de manière réversible ou irréversible, de sorte à enserrer l'une des zones de liaison de la paroi périphérique et assurer le maintien en position de l'organe de fixation sur ladite zone de liaison.

Selon une réalisation, l'organe de préhension est solidarisé structurellement à l'au moins un conduit de transfert par le serrage de moyens d'encerclement sur une portion d'accouplement dudit au moins un conduit de transfert.

Selon une réalisation, l'organe de préhension et l'organe de fixation sont reliés, l'un à l'autre, par une liaison mécanique directe.

Selon une réalisation, préalablement à l'assemblage de l'organe de préhension avec l'organe de fixation, un organe intermédiaire de liaison distinct dudit organe de préhension et dudit organe de fixation est solidarisé à l'organe de préhension ; cet organe intermédiaire de liaison étant ensuite solidarisé à l'organe de fixation pour garantir le positionnement dudit organe de préhension et de l'au moins un conduit de transfert qui lui est solidaire à proximité dudit organe de fixation et de la zone de liaison de la paroi périphérique correspondante.

Selon une réalisation, avant de solidariser structurellement n conduits de transfert, n étant un entier supérieur à 1, à l'organe de préhension, lesdits n conduits de transfert sont placés dans des sièges de réception formés sur une entretoise de cloisonnement de manière à empêcher le contact desdits n conduits de transferts les uns avec les autres et à éviter que l'un de ces n conduits de transfert ne soient écrasés sous l'effet de la contrainte de serrage. Selon une réalisation, une première série d'organes de préhension est solidarisée à une première série de portions d'accouplement disposées le long d'une première série de conduit(s) de transfert ; une première série d'organes de fixation, structurellement distincts et indépendants des organes de préhension, est fixée sur une première série de zones de liaison de la paroi périphérique sans affecter son étanchéité, puis chaque organe de préhension de la première série est assemblé de façon structurelle ou fonctionnelle sur un organe de fixation de la première série afin de guider la première série de conduit(s) de transfert à proximité de la paroi périphérique et jusqu'à un premier emplacement correspondant à la zone d'introduction ou de retrait d'un premier produit de transfert.

Dans ce cas, selon une première variante de réalisation, les organes de préhension de la première série sont solidarisés le long de la première série de conduit(s) de transfert de manière à définir, entre les portions d'accouplement de la première série, des intervalles de solidarisation prédéterminés ; les organes de fixation de la première série sont fixés sur la paroi périphérique de manière à définir, entre les zones de liaison de la première série, des intervalles de liaison prédéterminés et similaires aux intervalles de solidarisation ; de sorte que, après l'assemblage structurel ou fonctionnel des organes de préhension et des organes de fixation de la première série, la première série de conduit(s) de transfert définissent une première trajectoire coïncidant avec la trajectoire définie par la première série de zones de liaison sur la paroi périphérique.

Dans ce cas, selon une première variante de réalisation, les organes de préhension de la première série sont solidarisés le long de la première série de conduit(s) de transfert de manière à définir, entre les portions d'accouplement de la première série, des intervalles de solidarisation prédéterminés ; les organes de fixation de la première série sont fixés sur la paroi périphérique de manière à définir, entre les zones de liaison de la première série, des intervalles de liaison prédéterminés et inférieurs aux intervalles de solidarisation ; de sorte que, après l'assemblage structurel ou fonctionnel des organes de préhension et des organes de fixation de la première série, la première série de conduit(s) de transfert définissent une première trajectoire distincte de la trajectoire définie par la première série de zones de liaison sur la paroi périphérique. Une telle variante permet de définir précisément la trajectoire de la première série de conduit(s) de transfert, tout en limitant le nombre d'organes de guidage à utiliser.

Selon une réalisation, une deuxième série d'organes de préhension sont solidarisés le long d'un ou plusieurs autre(s) conduit(s) de transfert ; une deuxième série d'organes de fixation, structurellement distincts et indépendants des organes de préhension sont fixés sur une deuxième série de zones de liaison de la paroi périphérique sans affecter sa continuité structurelle ou son étanchéité et en définissant une deuxième trajectoire prédéterminée ; puis chaque organe de préhension de la deuxième série est assemblé de façon structurelle ou fonctionnelle sur un organe de fixation de ladite deuxième série afin de guider la deuxième série de conduit(s) de transfert à proximité de la paroi périphérique et jusqu'à un deuxième emplacement correspondant à la zone d'introduction ou de retrait d'un deuxième produit de transfert.

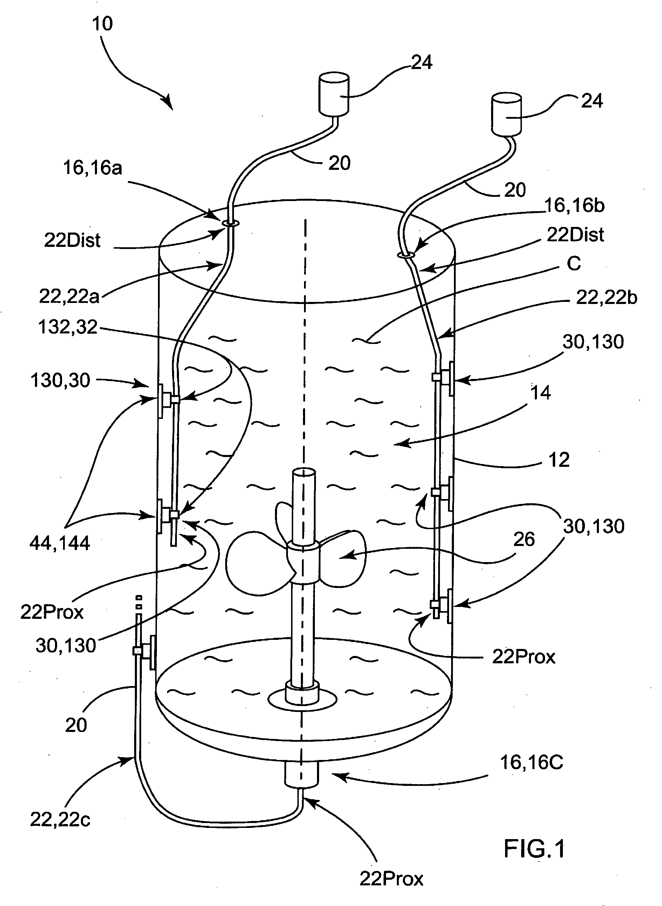

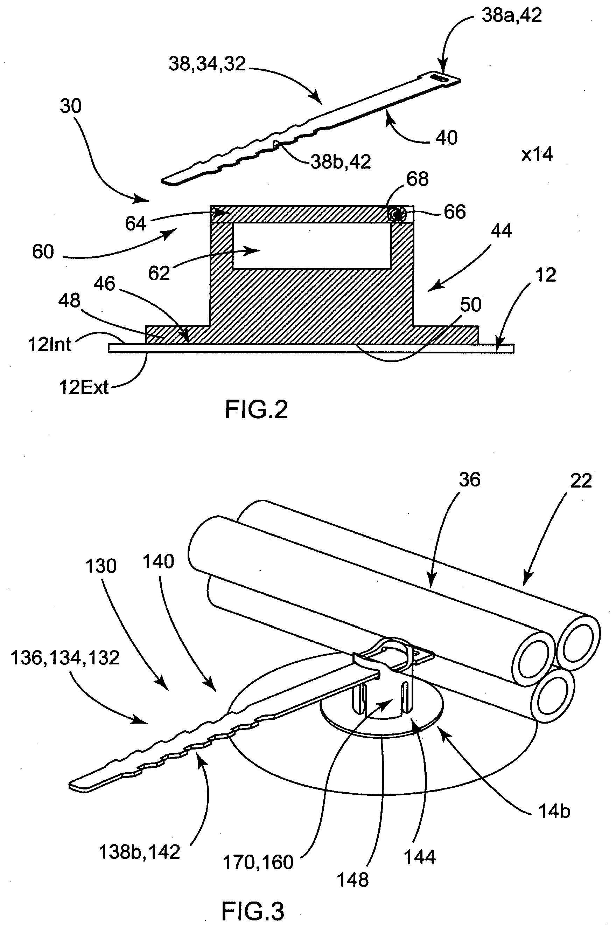

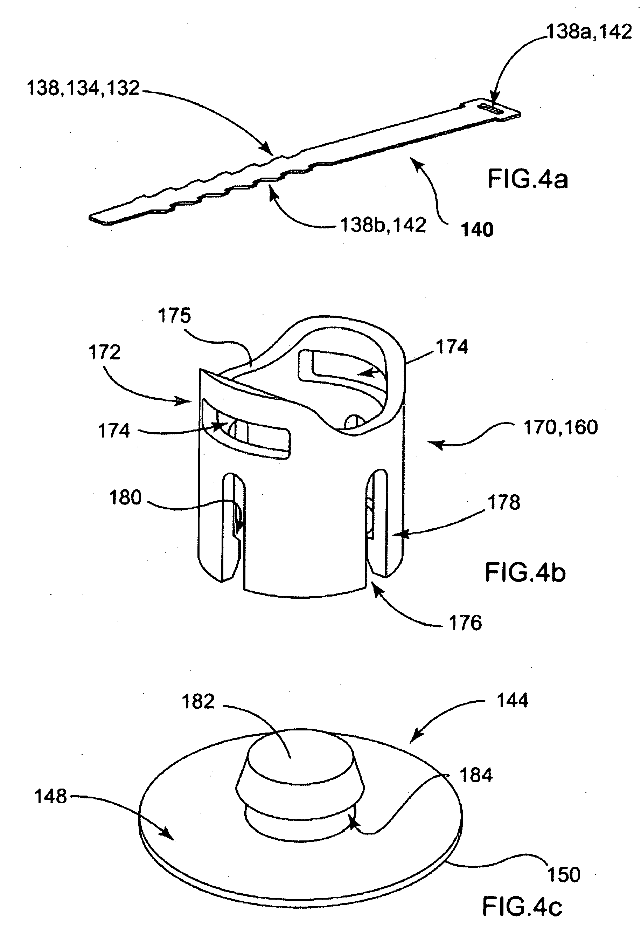

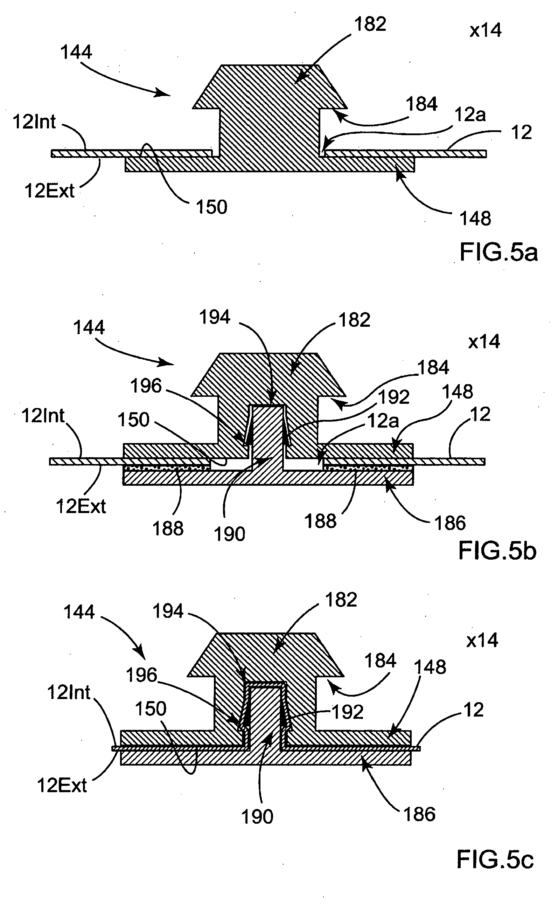

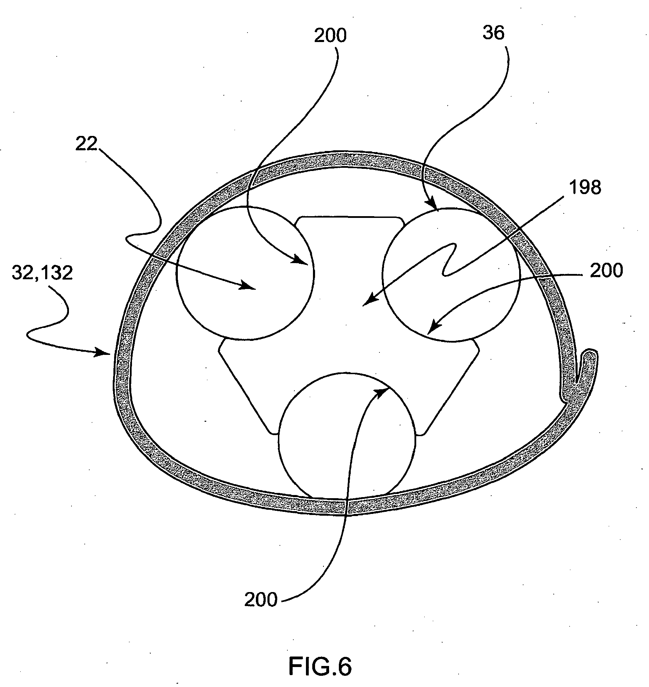

D'autres caractéristiques et avantages de l'invention ressortiront clairement de la description qui en est faite ci-après, à titre indicatif et nullement limitatif, en référence aux dessins annexés, dans lesquels :
▪ La figure 1 est une vue générale, en perspective, d'un conteneur selon l'invention comprenant une paroi périphérique définissant un espace intérieur de réception du contenu biopharmaceutique, deux conduits de transfert et plusieurs éléments de guidage fixés contre la face interne de la paroi périphérique pour maintenir les conduits de transferts selon deux trajectoires prédéterminées et jusqu'à deux emplacements distincts à l'intérieur de l'espace interne de réception ;
▪ La figure 2 est une vue de détail, en coupe transversale, d'un premier mode de réalisation d'un élément de guidage appartenant au conteneur selon l'invention, dans lequel l'élément de guidage est soudé sur la face interne de la paroi périphérique et composé d'un organe de préhension et d'un organe de fixation destinés à être solidarisés directement et structurellement l'un avec l'autre ;
▪ La figure 3 est une vue de détail, en perspective, d'un deuxième mode de réalisation d'un élément de guidage appartenant au conteneur selon l'invention, cet élément de guidage recevant trois conduits de transfert et étant composé d'un organe de fixation, d'un organe intermédiaire de liaison et d'un organe de préhension constitué par une boucle de serrage et de blocage ;
▪ La figure 4a est une vue de détail, en perspective, de l'organe de préhension appartenant à l'élément de guidage de la figure 3, dans lequel cet organe de préhension est formé par une boucle de serrage et de blocage ;
▪ La figure 4b est une vue de détail, en perspective, de l'organe intermédiaire de liaison appartenant à l'élément de guidage de la figure 3, dans lequel cet organe intermédiaire de liaison comporte des moyens de solidarisation à l'organe de fixation par enclipsage ;
▪ La figure 4c est une vue de détail, en perspective, de l'organe de fixation appartenant à l'élément de guidage de la figure 3, dans lequel cet organe de fixation comporte une base de fixation dotée d'une face plate et présentant un tenue intrinsèque suffisante pour lui éviter toute déformation structurelle, en l'absence de sollicitation extérieure ;
▪ La figure 5a est une vue de détail, en coupe transversale, d'une première variante de réalisation d'un conteneur selon l'invention dans laquelle, d'une part, la paroi périphérique présente une ouverture de fixation et, d'autre part, l'organe de fixation est soudé autour de l'ouverture de fixation, sur la face externe de la paroi périphérique, afin d'assurer l'étanchéité de cette paroi périphérique tout en autorisant l'assemblage dudit organe de fixation avec l'organe intermédiaire de fixation placé dans l'espace interne de réception du conteneur ;
▪ La figure 5b est une vue de détail, en coupe transversale, d'une deuxième variante de réalisation d'un conteneur selon l'invention dans laquelle, premièrement, la paroi périphérique présente une ouverture de fixation, deuxièmement, l'organe de fixation est positionné contre la face interne de la paroi périphérique et, troisièmement, une contre pièce de fixation est positionnée contre la face externe de la paroi périphérique, autour de l'ouverture de fixation, de manière à assurer l'étanchéité de la paroi périphérique tout en maintenant l'organe de fixation en position ;
▪ La figure 5c est une vue de détail, en coupe transversale, d'une troisième variante de réalisation d'un conteneur selon l'invention, dans laquelle, premièrement, la paroi périphérique présente une continuité structurelle, deuxièmement, l'organe de fixation est positionné contre la face interne de la paroi périphérique et, troisièmement, une contre pièce de fixation est positionnée contre la face externe de la paroi périphérique et coopère avec l'organe de fixation de manière à le maintenir en position contre la paroi périphérique du conteneur ;
▪ La figure 6 est une représentation symbolique, en coupe transversale, de trois conduits de transfert enserrés par un élément de préhension appartenant à un conteneur selon l'invention et dans lequel une entretoise de cloisonnement présente trois sièges de réception dans lesquels reposent les conduits de transfert.

Un conteneur 10 selon l'invention est décrit en référence à la figure 1.

Le conteneur 10 en question est destiné à recevoir un contenu biopharmaceutique C en vue de son conditionnement, de son mélange, de la réalisation d'une bioréaction, ou de toute autre réaction chimique.

Le contenu biopharmaceutique C peut contenir un ou plusieurs composants susceptibles de se trouver en phase liquide, gazeuse, ou bien solide, tel que de la poudre. Le cas échéant, le conteneur 10 peut également contenir des cellules, des micro-organismes, etc.

Le conteneur 10 selon la réalisation de la figure 1 présente une géométrie de révolution autour d'une axe géométrique A-A dirigé dans la direction verticale lorsque le conteneur est en position d'utilisation. Il convient toutefois de signaler que ce conteneur 10 pourrait présenter d'autres formes, notamment parallélépipédique ou autre.

Le conteneur 10 comprend, en premier lieu, une paroi périphérique 12 présentant une face interne 12_{INT} et une face externe 12_{EXT}. Cette paroi périphérique 12 peut être formée à partir d'un seul tronçon ou bien à partir de plusieurs tronçons solidarisés les uns aux autres par soudage, collage ou tout autre procédé analogue assurant une étanchéité fonctionnelle entre les différents tronçons.

La paroi périphérique 12 est, par ailleurs, refermée sur elle-même afin de former une chambre interne de réception 14 susceptible de recevoir une certaine quantité du contenu biopharmaceutique C.

Selon une réalisation, le conteneur 10 est à usage unique.

Il peut, par ailleurs, selon différentes variantes de réalisation, avoir une capacité allant de 10 litres à 5.000 litres en fonction des besoins et des applications requises.

Dans la suite de la description, le conteneur 10 est présenté en position d'utilisation, c'est-à-dire lorsqu'il reçoit le contenu biopharmaceutique C. Les mots « vertical », « horizontal », « supérieur » et « inférieur » se réfèrent donc aux positions spatiales des éléments lorsque le conteneur 10 est dans une position lui permettant de fonctionner. Toutefois, il est entendu que le conteneur 10 peut occuper d'autres positions ou avoir d'autres états, par exemple lorsqu'il est lui-même conditionné ou qu'il n'est pas en fonctionnement.

D'autre part, la direction « axiale » se réfère à ce qui est orienté au moins sensiblement dans la direction de l'axe géométrique A-A alors que la direction « radiale » se rapporte à ce qui est orienté dans une direction sensiblement issue de l'axe géométrique A-A et s'étendant dans un plan perpendiculaire à cet axe géométrique A-A. Par ailleurs, un plan « transversal » est un plan sensiblement perpendiculaire à l'axe géométrique A-A.

En outre, les termes « intérieur » et « extérieur » se réfèrent respectivement à ce qui se trouve dans et en dehors du conteneur 10.

Le conteneur 10 comporte également au moins un port 16 coopérant avec un orifice aménagé dans la paroi périphérique 12 du conteneur 10 et susceptible d'être obturé ou bien ouvert à tout moment pour permettre, notamment, l'introduction ou le retrait de produits qualifiés, de manière générale, de produits de transfert 20.

Il convient de signaler qu'un tel produit de transfert 20 peut correspondre à tout composant se trouvant ou étant destiné à se trouver en phase liquide, gazeuse, ou bien solide dans l'espace interne de réception 14 du conteneur 10. Plus particulièrement, le produit de transfert 20 peut, dans certains cas, correspondre au contenu biopharmaceutique C lui-même lorsqu'il s'agit de l'introduire ou de le retirer du conteneur 10. Il peut également s'agir de réactifs destinés à être ajoutés dans le conteneur 10 pour réaliser la réaction chimique permettant d'aboutir à la formation du contenu biopharmaceutique C ou bien, à l'inverse, il peut s'agir d'un produit de cette réaction chimique devant être retiré du conteneur 10 afin de finaliser la formation du contenu biopharmaceutique C. Le produit de transfert 20 peut aussi être un gaz introduit dans le conteneur 10 pour aérer le produit biopharmaceutique C.

Dans la réalisation de la figure 1, le conteneur 10 comprend trois ports 16 distincts.

À titre exemplatif et non limitatif, le premier d'entre eux peut constituer un port d'introduction 16a permettant d'introduire un ou plusieurs produit(s) de transfert 20 - correspondant à des réactifs - depuis l'extérieur et vers la chambre interne de réception 14. Le deuxième est un port d'extraction 16b destiné à retirer un ou plusieurs produit(s) de transfert 20 - correspondant à des produits de la réaction chimique - depuis la chambre interne de réception 14 et vers l'extérieur. Enfin, le troisième est un port de vidange 16c permettant de vider totalement le conteneur 10 du contenu biopharmaceutique C.

Dans cet exemple, les ports d'introduction 16a et d'extraction 16b sont placés à l'extrémité supérieure du conteneur 10 tandis que le port de vidange 16c est positionné à l'extrémité inférieur du conteneur 10.

Aux ports 16 peuvent être associés, en communication fluidique et avec une connexion étanche et, le cas échéant, amovible des conduits de transfert 22 destinés à assurer le transport des produits de transfert entre la chambre interne de réception 14 et l'extérieur du conteneur 10. Qu'ils soient situés à l'extérieur du conteneur 10 ou bien dans la chambre interne de réception 14, ces conduits de transfert 22 présentent une portion extrême proximale 22_{PROX} ouverte sur la chambre interne de réception 14 et une portion extrême distale 22_{DIST} ouverte sur l'extérieur du conteneur 10. Ces portions extrêmes proximale 22_{PROX} et distale 22_{DIST}, lorsqu'ils sont connectés à un port 16, sont généralement en connexion étanche et amovible avec ce dernier. La portion extrême distale 22_{DIST} du conduit de transfert 22 peut également se trouver en connexion étanche et, généralement, amovible avec un réservoir 24, une cuve, une poche ou analogue.

Le conteneur 10 présente ainsi deux conduits de transfert 22 situés à l'extérieur du contenant 10 et reliant deux réservoirs 24 aux ports d'introduction 16a et d'extraction 16b.

Le conteneur 10 comporte par ailleurs un conduit de transfert 22, dénommé conduit de transfert à fonction d'introduction 22a, situé dans l'espace interne de réception 14 et reliant le port d'introduction 16a à un emplacement d'introduction au niveau duquel peut être introduit le produit de transfert.

Le conteneur 10 comporte aussi un conduit de transfert 22, dénommé conduit de transfert à fonction d'extraction 22b, situé dans l'espace interne de réception 14 et reliant le port d'extraction 16b à un emplacement d'extraction distinct de l'emplacement d'introduction notamment en ce qu'ils ne sont pas situés à la même hauteur dans l'espace interne d'introduction.

Le conteneur 10 comporte en outre un conduit de transfert 22, dénommé conduit de transfert à fonction de vidange 22c, situé à l'extérieur du contenant 10 et reliant le port de vidange 16c à une zone de vidange (non représentée).

Il convient de signaler que le produit de transfert 20 susceptible d'être introduit dans le conteneur 10 peut correspondre à toute sorte de réactif, tel qu'un produit biopharmaceutique, un milieu de culture, des cellules biologiques, du gaz et notamment de l'oxygène, etc. De la même manière, le produit de transfert 20 destiné à être extrait du conteneur 10 peut correspondre au produit biopharmaceutique C en tant que tel ou bien à tout autre élément situé à un emplacement prédéterminer à l'intérieur du conteneur 10. Il en est de même pour le produit de transfert 20 destiné à être vidangé.

Selon la réalisation de la figure 1, le conteneur 10 comprend également des moyens de mélange 26 situé à l'intérieur de la chambre interne de réception 14 et capable de mélanger l'ensemble du contenu biopharmaceutique C ou bien une partie seulement des composants de ce contenu biopharmaceutique C. Il convient toutefois de signaler que le conteneur 10 selon l'invention peut ne pas comprendre de tels moyens de mélange 26.

À l'inverse, ledit conteneur 10 peut également comprendre d'autres éléments - tels que capteurs, système de chauffage, de refroidissement, etc. - susceptible d'améliorer la qualité de la réaction chimique ayant lieu dans la chambre interne de réception 14 ou bien, plus simplement, le conditionnement du contenu biopharmaceutique.

Le conteneur 10 comprend également au moins un élément de guidage 30,130.

Plus particulièrement, le conteneur 10 illustré par la figure 1 comprend une première série de deux éléments de guidage 30 ; 130.

Ces deux éléments de guidage 30 ; 130 de la première série sont supportés par la face interne 12_{INT} de la paroi périphérique 12 et maintiennent le conduit de transfert à fonction d'introduction 22a à proximité de cette paroi périphérique 12. Les deux éléments de guidage 30; 130 de la première série qui sont situés à des hauteurs distinctes permettent donc d'imposer une trajectoire d'introduction prédéterminée, en l'espèce verticale, entre les portions extrêmes proximale 22_{PROX} et distale 22_{DIST} du conduit de transfert à fonction d'introduction 22a.

D'autre part, l'un des deux éléments de guidage 30 ; 130, maintient la portion extrême proximale 22_{PROX} du conduit de transfert à fonction d'introduction 22a de sorte à le guider jusqu'à un emplacement prédéterminé correspondant à la zone d'introduction du produit de transfert 20.

Grâce à cet agencement, la zone d'introduction de ce produit de transfert 20 est précisément déterminée, ce qui peut éventuellement permettre d'améliorer les paramètres de la réaction chimique en cours dans la chambre interne de réception 14 du conteneur 10.

Le conteneur 10 de la figure 1 comprend également une deuxième série de trois éléments de guidage 30 ; 130.

Ces trois éléments de guidage 30 ; 130 de la deuxième série sont supportés par la face interne 12_{INT} de la paroi périphérique 12 et maintiennent le conduit de transfert à fonction de retrait 22b à proximité de cette paroi périphérique 12. Les trois éléments de guidage 30 ; 130 de la deuxième série qui sont situés à des hauteurs distinctes permettent donc d'imposer une trajectoire d'introduction prédéterminée, en l'espèce verticale, entre les portions extrêmes proximale 22_{PROX} et distale 22_{DIST} du conduit de transfert à fonction de retrait 22b. D'autre part, l'un des trois éléments de guidage 30 ; 130, maintient la portion extrême proximale 22_{PROX} du conduit de transfert à fonction de retrait 22b de sorte à le guider jusqu'à un emplacement prédéterminé correspondant à la zone d'extraction du produit de transfert 20. La zone d'extraction de ce produit de transfert 20, potentiellement différente de la zone d'introduction décrite précédemment, est également précisément déterminée.

Le conteneur de la figure 1 comporte, en outre, une troisième série composée d'un unique élément de guidage 30 ; 130.

Cet élément de guidage 30; 130 est fixé contre la face externe 12_{EXT} de la paroi périphérique et maintien le conduit de transfert à fonction de vidange 22c à l'extérieur du conteneur 10 mais à proximité de sa paroi périphérique 12. L'unique élément de guidage 30 ; 130 impose, par ailleurs, une trajectoire de vidange prédéterminée, entre les portions extrêmes proximale 22_{PROX} et distale 22_{DIST} du conduit de transfert à fonction de vidange 22c.

Il convient de souligner que les trajectoires imposées par les première et deuxième séries d'éléments de guidage 30, 130 sont ici verticales mais pourrait également être courbes, inclinées, etc. selon les besoins de la réaction et les contraintes d'encombrement dans la chambre interne de réception 14.

Un premier exemple de réalisation d'un élément de guidage 30 susceptible d'être employé dans le conteneur 10 selon l'invention est illustré par la figure 2.

Cet élément de guidage 30 comporte, tout d'abord, un organe de préhension 32.

L'organe de préhension 32 est capable d'être solidarisé structurellement ou fonctionnellement aux conduits de transfert 22.

Plus particulièrement, selon la réalisation de la figure 2, cet organe de préhension 32 forme des moyens d'encerclement 34 capables d'enserrer une portion d'accouplement 36 des conduits de transfert 22.

Les moyens d'encerclement 34 s'apparentent donc, en l'espèce, à une lanière 38 présentant une extrémité dotée d'une boucle 38a dans laquelle peut être introduite l'autre extrémité de manière à former des moyens de serrage 40.

De cette façon, pour enserrer un ou plusieurs conduit(s) de transfert 22, il est possible de disposer la lanière 38 autour de la portion d'accouplement 36 dudit ou desdits conduit(s) de transfert 22 et d'introduire l'extrémité de cette lanière 38 dans la boucle 38a opposée jusqu'à ce que le ou les conduit(s) de transfert 22 soi(en)t enserré(s). Les moyens d'encerclement 34 peuvent ainsi être maintenus serrés ou bien avec une certaine lâcheté contre les portions d'accouplement 36 des conduits de transfert 22. Ils sont toutefois maintenus de manière à ne pas engendrer d'écrasement de ces portions d'accouplement 36.

De tels moyens d'encerclement 34 ont pour avantage de s'adapter aux dimensions du ou des conduit(s) de transfert 22 à enserrer tout en permettant de contrôler les risques d'écrasement dudit ou desdits conduit(s) de transfert 22.

Les moyens d'encerclement 34 présentent également une série d'encoches 38b réparties sur la longueur de la lanière 38 et capables de coopérer avec la boucle 38a pour constituer des moyens de blocage 42 lorsque les moyens d'encerclement 34 sont en position serrés contre les portions d'accouplement 36 des conduits de transfert 22.

Ainsi, lorsque les moyens de serrage 40 sont ajustés contre la portion d'accouplement 36 du ou des conduit(s) de transfert 22, les moyens de blocage 42 permettent de les maintenir en position et donc de conserver les moyens d'encerclement 34 contre ledit ou lesdits conduit(s) de transfert 22.

De manière alternative, il convient de signaler que les moyens d'encerclement 34 peuvent également être formés à partir d'une boucle (non représentée) refermée sur elle-même et présentant une élasticité suffisante pour constituer simultanément les moyens de serrage 40 et les moyens de blocage 42. Il est ainsi possible d'étirer la boucle pour y introduire le ou les conduit(s) de transfert 22 et, ensuite, de la laisser se resserrer - par élasticité - sur la portion d'accouplement 36 du ou des conduit(s) de transfert 22 afin de garantir leur maintien en position, sans qu'ils ne plient sous l'effet de la contrainte de serrage.

D'autre part, selon d'autres modes de réalisation encore, l'organe de préhension 32 peut être réalisé par tout autre moyen, connu de l'homme du métier.

L'élément de guidage 30 de la figure 2 comporte également un organe de fixation 44.

Cet organe de fixation 44 est destiné à venir se fixer sur n'importe quelle zone de liaison 46 de la paroi périphérique 12 sans affecter son étanchéité.

Plus particulièrement, selon ce mode de réalisation, l'organe de fixation 44 comporte une base de fixation 48 dotée d'une tenue intrinsèque suffisante pour éviter toute déformation structurelle, en l'absence de sollicitation extérieure autre que celle de la gravité. Cette tenue intrinsèque permet ainsi à la base de fixation 46 d'être manipulée plus aisément et de faciliter sa fixation sur la zone de liaison 46 de la paroi périphérique 12. Mais à l'inverse, il pourrait être prévu que la base de fixation 46 présente une certaine flexibilité, afin d'autoriser sa fixation sur d'éventuelles zones courbes de la paroi périphérique 12.

D'autre part, selon la réalisation de la figure 2, la base de fixation 48 présente une face plate 50 pouvant être solidarisée directement contre la paroi périphérique 12, notamment par soudage. Cette fixation par soudage présente l'avantage d'être simple et de ne pas nécessiter d'endommager la continuité structurelle de la paroi périphérique 12. De manière alternative, la fixation de la face plate 50 contre la zone de liaison 46 de la paroi périphérique 12 pourrait aussi être réalisée par collage ou tout autre procédé analogue présentant des avantages similaires au soudage.

Selon l'invention, l'organe de préhension 32 et l'organe de fixation 44 sont structurellement distincts et indépendants l'un de l'autre.

L'élément de guidage 30 présente des moyens d'assemblage réciproques 60.

Ces moyens d'assemblage réciproques 60 permettent d'assurer l'assemblage structurel de l'organe de préhension 32, lorsque ce dernier est solidaire d'un ou plusieurs conduit(s) de transfert 22, avec l'organe de fixation 44, lorsque ce dernier est fixé sur la zone de liaison 46 de la paroi périphérique 12.

Ainsi les moyens d'assemblage 60 garantissent le positionnement de l'organe de préhension 32 à proximité de l'organe de fixation 44 et de la zone de liaison 46 de la paroi périphérique 12 correspondante.

Selon la réalisation de la figure 2, les moyens d'assemblage réciproques 60 sont formés par une ouverture de réception 62 agencée dans le corps de l'organe de fixation 44 sur laquelle vient se refermer un rabat de fermeture 64.

Plus particulièrement, le rabat de fermeture 64 est capable de se déplacer en rotation autour d'un axe géométrique 66 afin de passer d'une position ouverte dans laquelle les moyens d'encerclement 34 sont libres, à une position fermée dans laquelle le rabat de fermeture 64 bloque une portion de ces moyens d'encerclement 34 à l'intérieur de l'ouverture de réception 62. Le rabat de fermeture 64 peut alors être maintenu dans la position fermée par le biais d'un moyen ressort 68 comme dans l'exemple de la figure 2 ou bien par toute autre moyen analogue.

De cette façon, la lanière 38 est bloquée à l'intérieur de l'ouverture de réception 62 et l'organe de préhension 32 reste en position à proximité de l'organe de fixation 44.

De tels moyens d'assemblage réciproques 60 facilitent la mise en place des conduits de transfert 22 dans la chambre interne de réception 14 puisque l'opérateur peut réaliser, d'abord, la préhension des conduit(s) de transfert 22 avec l'organe de préhension 32 et la mise en place de l'organe de fixation 44 contre la paroi périphérique 12 et, ensuite, l'assemblage de l'organe de préhension 32 et de l'organe de fixation 44. Les risques de contamination de l'espace interne de réception 14 et les défauts susceptibles d'être générés lors de cette opération d'assemblage sont ainsi largement réduits. Par ailleurs, solidariser l'organe de préhension 32 avec le ou les conduit(s) de préhension 22 avant son association avec l'organe de fixation 44 permet d'être plus précis et donc de garantir un positionnement adéquate du conduit(s) de transfert 22 dans la chambre interne de réception 14. De la même manière, solidariser l'organe de fixation 44 à la paroi périphérique 12 du conteneur 10 préalablement à son association avec l'organe de préhension 32 et le ou les conduit(s) de transfert simplifie et accélère cette étape du procédé de fabrication.

Un deuxième exemple de réalisation d'un élément de guidage 130 susceptible d'être employé dans le conteneur 10 selon l'invention est illustré par les figures 3, 4a, 4b et 4c.

Cet élément de guidage 130 comporte un organe de préhension 132 similaire à celui de l'exemple de réalisation de la figure 2.

Plus particulièrement, l'organe de préhension 132 est capable d'être solidarisé structurellement ou fonctionnellement aux conduits de transfert 22. Il forme des moyens d'encerclement 134 capables d'enserrer une portion d'accouplement 136 des conduits de transfert 22.

Les moyens d'encerclement 134 s'apparentent donc à une lanière 138 présentant une extrémité dotée d'une boucle 138a dans laquelle peut être introduite l'autre extrémité de manière à former des moyens de serrage 140.

De cette façon, pour enserrer un ou plusieurs conduit(s) de transfert 22, il est possible de disposer la lanière 138 autour de la portion d'accouplement 136 dudit ou desdits conduit(s) de transfert 22 et d'introduire l'extrémité de cette lanière 138 dans la boucle 38a opposée jusqu'à ce que le ou les conduit(s) de transfert 22 soi(en)t enserré(s). Les moyens d'encerclement 134 peuvent ainsi être maintenus serrés ou bien avec une certaine lâcheté contre les portions d'accouplement 36 des conduits de transfert 22. Ils sont toutefois maintenus de manière à ne pas engendrer l'écrasement de ces portions d'accouplement 136 sous l'effet des contraintes de serrage.

Les moyens d'encerclement 134 présentent également une série d'encoches 138b réparties sur la longueur de la lanière 38 et capables de coopérer avec la boucle 138a pour constituer des moyens de blocage 142 lorsque les moyens d'encerclement 134 sont en position serrés contre les portions d'accouplement 36 des conduits de transfert 22.

Ainsi, lorsque les moyens de serrage 140 sont ajustés contre la portion d'accouplement 136 du ou des conduit(s) de transfert 22, les moyens de blocage 142 permettent de les maintenir en position et donc de conserver les moyens d'encerclement 134 contre ledit ou lesdits conduit(s) de transfert 22.

Comme précédemment, il est entendu que l'organe de préhension 132 peut également être formé à partir d'une boucle refermée sur elle-même et présentant une élasticité suffisante pour constituer simultanément les moyens de serrage 140 et les moyens de blocage 142 ou bien à partir de toute autre réalisation.

L'élément de guidage 130 de la figure 3 comporte également un organe de fixation 144.

Cet organe de fixation 144 est destiné à venir se fixer sur n'importe quelle zone de liaison 146 de la paroi périphérique 12 sans affecter son étanchéité.

Plus particulièrement, selon ce mode de réalisation, l'organe de fixation 144 comporte une base de fixation 148 dotée d'une tenue intrinsèque suffisante pour éviter toute déformation structurelle, en l'absence de sollicitation extérieure. Cette tenue intrinsèque permet ainsi à la base de fixation 146 d'être manipulée plus aisément et de facilité sa fixation sur la zone de liaison 146 de la paroi périphérique 12. Mais à l'inverse, cette base de fixation 146 pourrait également présenter une certaine flexibilité, afin d'autoriser sa fixation sur d'éventuelles zones courbes de la paroi périphérique 12.

D'autre part, la base de fixation 148 présente une face plate 150 pouvant être solidarisée directement contre la paroi périphérique 12, notamment par soudage. Cette fixation par soudage présente l'avantage d'être simple et de ne pas nécessiter d'endommager la continuité structurelle de la paroi périphérique 12. La fixation de la face plate 150 contre la zone de liaison 146 de la paroi périphérique 12 pourrait toutefois également se faire par collage ou tout autre procédé analogue.

Selon l'invention, l'organe de préhension 132 et l'organe de fixation 144 sont structurellement distincts et indépendants l'un de l'autre.

Mais ces organes de préhension 132 et de fixation 144 sont aptes à être solidarisés fonctionnellement l'un à l'autre par l'intermédiaire de moyens d'assemblage réciproques 160 permettant d'assurer l'assemblage fonctionnel de l'organe de préhension 132, lorsque ce dernier est solidaire d'un ou plusieurs conduit(s) de transfert 22, avec l'organe de fixation 144, lorsque ce dernier est fixé sur la zone de liaison 46 de la paroi périphérique 12. Ainsi les moyens d'assemblage réciproques 160 garantissent le positionnement de l'organe de préhension 132 à proximité de l'organe de fixation 144 et de la zone de liaison 146 de la paroi périphérique 12 correspondante.

Plus particulièrement, selon le mode de réalisation de la figure, 3, ces moyens d'assemblage réciproques 160 sont en partie formés par un organe intermédiaire de liaison 170 distinct de l'organe de préhension 132 et de l'organe de fixation 144.

Cet organe intermédiaire de liaison 170 présente des moyens de solidarisation 172 à l'organe de préhension 132.

Notamment, selon la réalisation de la figure 3, les moyens de solidarisation 172 à l'organe de préhension 132 sont formés par deux boucles 174 à l'intérieur desquelles peuvent passer les moyens d'encerclement 134 avant d'enserrer le ou les conduit(s) de transfert. De tels moyens de solidarisation 172 à l'organe de préhension 132 ont l'avantage d'être simples à fabriquer et à utiliser.

En outre, ces moyens de solidarisation 172 à l'organe de préhension 132 constituent, entre les deux boucles 174, un berceau de réception 175 épousant la forme d'au moins un conduit(s) de transfert 22. Le berceau de réception 175 peut ainsi recevoir ledit au moins un tube(s) de transfert 22 pour faciliter sa solidarisation à l'organe intermédiaire de liaison 170 tout en limitant les risques d'écrasement de ce dernier.

L'organe intermédiaire de liaison 170 présente aussi des moyens de solidarisation 176 à l'organe de fixation 144.

Plus particulièrement, selon cette réalisation, l'organe intermédiaire de liaison 170 à l'organe de fixation 144 forme un élément femelle 178 doté d'un épaulement de maintien 180 présentant une certaine souplesse du fait de la géométrie de l'organe intermédiaire de liaison 170. Par ailleurs, l'organe de fixation 144 forme un élément mâle 182 doté d'un épaulement complémentaire 184 susceptible d'être inséré et maintenu dans l'élément femelle 178 par encliquetage.

Notamment, l'élément mâle 182 peut être introduit en force dans l'élément femelle 178 par déformation élastique de l'épaulement de maintien 180, puis pousser à l'intérieur de cet élément femelle 178 jusqu'à ce que l'épaulement complémentaire 184 dépasse l'épaulement de maintien 180. L'épaulement de maintien 180 reprend alors sa forme initiale et bloque ainsi l'épaulement complémentaire 184 dans l'élément femelle 178, bloquant par la même occasion l'élément mâle 182 dans ledit élément femelle 178.

Une telle liaison mécanique a pour effet d'aboutir à une solidarisation fonctionnelle, rapide et particulièrement efficace, de l'organe de préhension 132 avec l'organe de fixation 144. Les risques de contamination de la chambre interne de réception 14 lors de la mise en place du ou des conduit(s) de transfert 22 sont ainsi largement réduits. En outre, une telle liaison permet également d'obtenir, entre l'organe intermédiaire de liaison 170 et l'organe de fixation 144, une liaison pivot permettant de faciliter l'assemblage fonctionnel des organes de préhension 132 et de fixation 144 en évitant d'imposer une orientation obligatoire de l'organe de préhension 132 - et donc des tubes de transfert 22 - par rapport à l'organe de fixation 144. Elle permet finalement d'aboutir à un positionnement précis des conduits de transfert 22 et ainsi de garantir l'introduction ou le retrait des produits de transfert 20 à un emplacement prédéterminé.

Bien entendu, cette liaison mécanique et les moyens d'assemblage réciproques 160 pourraient également être réalisés différemment sans pour autant sortir du champ de l'invention. Il serait ainsi possible de réaliser toute autre forme de solidarisation par une liaison complète, une liaison rotule, une liaison rotule à doigt, etc.

Les figures 5a, 5b et 5c représentent plusieurs variantes de conteneur 10 selon l'invention dans lesquelles l'organe de fixation 144 et la paroi périphérique 20 coopèrent différemment.

Il convient tout d'abord de signaler que si ces variantes de réalisation sont illustrées sur la base du deuxième exemple de réalisation de l'élément de guidage 130, elles pourraient être mise en oeuvre *mutatis mutandis* dans le cadre du premier exemple de réalisation de l'élément de guidage 30.

Il est, d'abord, fait référence à la variante de réalisation de la figure 5a.

Selon cette variante de réalisation, la paroi périphérique 12 présente une ouverture de fixation 12a et l'organe de fixation 144 est soudé autour de cette ouverture de fixation 12a, par exemple sur la face externe 12_{EXT} de la paroi périphérique 12.

De cette manière, l'étanchéité vis-à-vis de l'extérieur est assurée et l'assemblage de l'organe de fixation 144 avec l'organe intermédiaire de liaison 170, placé dans l'espace interne de réception 14 du conteneur 10, reste possible.

Il est, ensuite, fait référence à la variante de réalisation de la figure 5b.

Selon cette deuxième variante, la paroi périphérique 12 présente également une ouverture de fixation 12a et l'organe de fixation 144 est composé, d'une part, d'une base de fixation 148 et, d'autre part, d'une contre-pièce de fixation 186.

La contre-pièce de fixation 186 est apte à coopérer avec la base de fixation 148 pour enserrer la paroi périphérique 12 et assurer le maintien de l'organe de fixation 144 autour de l'ouverture périphérique 12a. Pour ce faire, la contre-pièce de fixation 186 est soit collée par l'intermédiaire d'une colle 188, soit soudée comme précédemment (non représenté) contre la face externe 12_{EXT} de la paroi périphérique 12 ce qui permet de garantir l'étanchéité de l'espace interne de réception 14 vis-à-vis de l'extérieur du conteneur 10. Par ailleurs, la base de fixation 148 ainsi que la contre-pièce de fixation 186 présentent des profils géométriques complémentaires pouvant être assemblés mécaniquement l'un à l'autre pour assurer le maintien en position de la base de fixation 148 contre la zone de liaison 146 de la paroi périphérique 12.

Plus particulièrement, la contre pièce-pièce de fixation 186 présente, en l'espèce, un corps 190 s'étendant vers l'espace interne de réception 14 et pourvu d'un épaulement de maintien 192 présentant une certaine élasticité tandis que la base de fixation 148 présente une cavité 194 apte à recevoir le corps 190 et dotée d'un épaulement complémentaire 196. De cette manière, le corps 190 de la contre-pièce de fixation 186 peut être introduit à travers l'ouverture de fixation 12a et jusque dans la cavité 194 de la base de fixation 148 de sorte que l'épaulement de maintien 192 et l'épaulement complémentaire 196 assurent le maintien de la base de fixation 148 en position.

Cette deuxième variante de réalisation est avantageuse car elle permet d'obtenir un assemblage efficace de la base de fixation 148 avec la contre-pièce de fixation 186 dans la mesure où ces deux pièces peuvent entrer en contact structurel direct l'une avec l'autre. Par ailleurs, compte-tenu de l'élasticité de l'épaulement de maintien 192, la base de fixation 148 est associée à la contre-pièce de fixation 186 de manière réversible. Cependant, cette association pourrait également être réalisée de manière irréversible.

Il est maintenant fait référence à la variante de réalisation de la figure 5c.

Cette troisième variante de réalisation se différencie de celle de la figure 5b en ce que la paroi périphérique 12 ne présente pas d'ouverture de fixation 12a.

L'organe de fixation 144 est alors composé, d'une part, d'une base de fixation 148 et, d'autre part, d'une contre-pièce de fixation 186 apte à coopérer avec la base de fixation 148 pour enserrer la paroi périphérique 12 sans altérer sa continuité, son intégrité structurelle et donc son étanchéité.

Pour ce faire, la paroi périphérique 12 présente une certaine souplesse et une certaine élasticité, la contre-pièce de fixation 186 est agencée contre la face externe 12_{EXT} de la paroi périphérique 12 tandis que la base de fixation 148 est agencée contre la face interne 12_{INT} de cette paroi périphérique 12. En outre, la base de fixation 148 ainsi que la contre-pièce de fixation 186 présentent des profils géométriques complémentaires pouvant être assemblés mécaniquement l'un à l'autre pour assurer le maintien en position de la base de fixation 148.

Plus particulièrement, la contre pièce-pièce de fixation 186 présente un corps 190 s'étendant en direction de l'espace interne de réception 14, pourvu d'un épaulement de maintien 192 doté d'une certaine élasticité. La base de fixation 148 présente quant-à-elle une cavité 194 apte à recevoir le corps 190 sans que ce dernier ne traverse la paroi périphérique 12. Cette cavité 194 est également dotée d'un épaulement complémentaire 196 ce qui permet de maintenir l'épaulement de maintien 192 et, donc, la base de fixation 148 en position.

Cette troisième variante de réalisation est avantageuse car elle permet d'obtenir un assemblage efficace de la base de fixation 148 avec la contre-pièce de fixation 186 sans avoir à percer préalablement la paroi périphérique 12. Par ailleurs, compte-tenu de l'élasticité de l'épaulement de maintien 192, la base de fixation 148 est associée à la contre-pièce de de fixation 186 de manière réversible. Cependant, cette association pourrait également être réalisée de manière irréversible.

Selon une réalisation, le conteneur 10 selon l'invention comprend également au moins une entretoise de cloisonnement 198 telle que représentée sur la figure 6.

Plus particulièrement, cette entretoise de cloisonnement 198 comporte une pluralité de sièges de réception 200 - en l'occurrence trois - capables de recevoir et de maintenir en position les conduits de transferts 22 en évitant que ces derniers n'entrent en contact les uns avec les autres.

Ces sièges de réception 200 sont régulièrement répartis sur le pourtour de l'entretoise de cloisonnement 198 afin que les conduits de transfert 22 soient soumis à dés contraintes de serrage sensiblement identiques.

Cela permet ainsi de limiter les risques d'écrasement de l'un de ces conduits de transfert 22 lors de la mise en place ou lors de l'utilisation de l'organe de préhension 32, 132.

Le procédé de réalisation d'un conteneur 10 selon l'invention est maintenant décrit en détail.

Ce procédé comprend une succession d'étapes consistant, en premier lieu, à disposer d'une paroi périphérique 12 destinée à être refermée sur elle-même pour définir une chambre interne de réception 14 capable de recevoir le contenu biopharmaceutique C.

Il convient ensuite, d'une part, de solidariser structurellement l'un des conduits de transfert 22 à l'un des organes de préhension 32 ; 132 et, d'autre part, de fixer l'un des organes de fixation 44, 144 sur n'importe quelle zone de liaison prédéterminée de la paroi périphérique 12 sans affecter son étanchéité.

Selon l'invention, il convient - après ces opérations - d'assembler de façon structurelle ou fonctionnelle ledit organe de préhension 32 ; 132 avec ledit organe de fixation 44, 144 afin de former l'un des éléments de guidage permettant de garantir le positionnement du conduit de transfert 22 à proximité dudit organe de fixation 44 ; 144 et, donc, de la zone de liaison 46 ; 146 sur laquelle il est fixé.

Dans le cas d'un conteneur 10 tel que décrit en référence à la figure 2, les organes de préhension 32 et de fixation 44 sont solidarisés structurellement et directement l'un à l'autre.

Dans le cas d'un conteneur 10 tel que décrit en référence aux figures 3, 4a, 4b et 4c, il convient, pour assurer l'assemblage fonctionnel de l'organe de préhension 132 avec l'organe de fixation 144, de solidariser structurellement l'organe intermédiaire de liaison 170 avec l'organe de préhension 132 avant de solidariser structurellement cet organe intermédiaire de liaison 170 avec l'organe de fixation 144.

D'autre part, lorsque le conteneur 10 présente une série d'éléments de guidage 30 ; 130 destinés à soutenir un ou plusieurs conduits de transfert le long d'une même trajectoire, le procédé selon l'invention consiste à solidariser une série d'organes de préhension 32 ; 132 le long du ou des conduit(s) de transfert 22. Il convient par ailleurs de fixer une série d'organes de fixation 44 ; 144, structurellement distincts et indépendants des organes de préhension 32 ; 132, sur une série de zones de liaison 46 ; 146 appartenant à la face interne 12_{INT} ou à la face externe 12_{EXT} de la paroi périphérique 12.

Puis, chaque organe de préhension 32 ; 132 est ensuite assemblé de façon structurelle ou fonctionnelle sur l'organe de fixation 44 ; 144 correspondant afin de guider les conduit(s) de transfert 22 à proximité de la paroi périphérique 12 et jusqu'à un emplacement correspondant par exemple à la zone d'introduction ou de retrait d'un produit de transfert 20.

Il est à noter que les organes de préhension 32 ; 132 peuvent alors être solidarisés le long des conduit(s) de transfert 22 de manière à définir, entre les portions d'accouplement 36 ; 136, des intervalles de solidarisation de même longueur que les intervalles de liaison définis entre les zones de liaison 46 ; 146. Ainsi, après l'assemblage structurel ou fonctionnel des organes de préhension 32 ; 132 et des organes de fixation 44 ; 144, le ou les conduit(s) de transfert 22 définissent une trajectoire coïncidant avec celle définie par les zones de liaison 46 ; 146 sur la paroi périphérique 12.

À l'inverse, les organes de préhension 32 ; 132 peuvent également être solidarisés le long du ou des conduit(s) de transfert 22 de manière à définir, entre les portions d'accouplement, des intervalles de solidarisation différents des intervalles de liaison définis entre les zones de liaison 46 ; 146. De cette manière, après l'assemblage structurel ou fonctionnel des organes de préhension 32 ; 132 et des organes de fixation 44 ; 144, le ou les conduit(s) de transfert définissent une trajectoire distincte de celle formée par les zones de liaison 46 ; 146 sur la paroi périphérique 12.

Une telle variante permet ainsi de définir précisément la trajectoire du ou des conduits de transfert 22, tout en limitant le nombre d'organes de guidage 30 ; 130 à utiliser.

## Revendications

1. Conteneur (10) pour la réception d'un contenu biopharmaceutique (C) sous forme liquide, fluide ou bien gazeuse, comprenant :
une paroi périphérique (12) refermée sur elle-même et définissant une chambre interne de réception (14) capable de recevoir le contenu biopharmaceutique (C) ;
au moins un conduit de transfert (22) présentant une portion extrême proximale (22_{PROX}) ouverte sur la chambre interne de réception (14) et une portion extrême distale (22_{DIST}) ouverte sur l'extérieur du conteneur (10) de manière à autoriser le transport d'un produit de transfert (20) entre ladite chambre interne de réception (14) et l'extérieur dudit conteneur (10) ;
au moins un élément de guidage (30 ; 130) apte à être supporté par la paroi périphérique (12) et à maintenir le au moins un conduit de transfert (22) en position à proximité de cette paroi périphérique (12) de manière à imposer une trajectoire prédéterminée entre la portion extrême proximale (22_{PROX}) et la portion extrême distale (22_{DIST}) ;
dans lequel le au moins un élément de guidage (30 ; 130) comporte, d'une part, un organe de préhension (32 ; 132) capable d'être solidarisé structurellement audit au moins un conduit de transfert (22) et, d'autre part, un organe de fixation (44 ; 144) apte à se fixer sur n'importe quelle zone de liaison (46 ; 146) prédéterminée de la paroi périphérique (12) sans affecter son étanchéité ;
**caractérisé en ce que** l'organe de préhension (32 ; 132) et l'organe de fixation (44 ; 144) sont structurellement distincts et indépendants l'un de l'autre et **en ce que** l'élément de guidage (30 ; 130) présente des moyens d'assemblage réciproques (60 ; 160) permettant d'assurer l'assemblage structurel ou fonctionnel de l'organe de préhension (32 ; 132), lorsque ce dernier est solidaire de l'au moins un conduit de transfert (22), avec l'organe de fixation (44 ; 144), lorsque ce dernier est fixé sur la zone de liaison (46 ; 146), pour garantir le positionnement dudit organe de préhension (32 ; 132) à proximité dudit organe de fixation (44 ; 144) et de la zone de liaison (46 ; 146) de la paroi périphérique (12) correspondante.

2. Conteneur (10) selon la revendication 1, dans lequel l'organe de fixation (44 ; 144) présente une base de fixation (48 ; 148) dotée d'une tenue intrinsèque suffisante pour éviter toute déformation structurelle, en l'absence de sollicitation extérieure.

3. Conteneur (10) selon la revendication 2, dans lequel la base de fixation (48 ; 148) présente une face plate (50 ; 150) apte à être fixée sur l'une des zones de liaison (46 ; 146) de la paroi périphérique (12) par un procédé de fixation irréversible tel que le soudage, le collage, ou analogue.

4. Conteneur (10) selon la revendication 2, dans lequel la base de fixation (48 ; 148) et une contre-pièce de fixation (186) sont aptes à être positionnées de part et d'autre de la paroi périphérique (12) de manière à enserrer l'une des zones de liaison (46 ; 146) de la paroi périphérique (12) et à assurer le maintien en position de l'organe de fixation (44 ; 144) sur ladite zone de liaison (46 ; 146).

5. Conteneur (10) selon la revendication 4, dans lequel :
- soit la base de fixation (48 ; 148) et la contre-pièce de fixation (186) présentent des profils géométriques complémentaires pouvant être assemblés mécaniquement l'un à l'autre, de manière réversible ou irréversible, sans traverser la paroi périphérique (12) ni altérer la continuité structurelle de cette dernière ;
- soit la paroi périphérique (12) présente au moins une ouverture de fixation (12a), l'une de la base de fixation (48 ; 148) et de la contre-pièce de fixation (186) étant apte à être fixée autour de l'ouverture de fixation (12a) de manière à assurer l'étanchéité de la chambre interne de réception (14) définit par la paroi périphérique (12), l'autre de ladite base de fixation (48 ; 148) et de ladite contre-pièce de fixation (186) étant apte à traverser l'ouverture de fixation (12a), la base de fixation (48 ; 148) et la contre-pièce de fixation (186) présentant des profils géométriques complémentaires pouvant d'être assemblés mécaniquement l'un à l'autre, de manière réversible ou irréversible.

6. Conteneur (10) selon l'une quelconque des revendications 1 à 5, dans lequel l'organe de préhension (32 ; 132) est formé par des moyens d'encerclement (34 ; 134) aptes à enserrer une portion d'accouplement (36 ; 136) du au moins un conduit de transfert (22).

7. Conteneur (10) selon la revendication 6, dans lequel les moyens d'encerclement (34 ; 134) présentent des moyens de serrage (40 ; 140) aptes à enserrer l'au moins un conduit de transfert (22) en s'ajustant à ses dimensions ainsi que des moyens de blocage (42 ; 142) en position serrée aptes à bloquer les moyens de serrage (40 ; 140) lorsque les moyens d'encerclement (34 ; 134) sont ajustés aux dimensions de l'au moins un conduit de transfert (22) sans l'écraser ou le plier sous l'effet de la contrainte de serrage et/ou dans lequel les moyens d'encerclement (34 ; 134) présentent une élasticité leur permettant d'enserrer le au moins un conduit de transfert (22) sans l'écraser ou le plier sous l'effet de la contrainte de serrage.

8. Conteneur (10) selon l'une quelconque des revendications 1 à 7, dans lequel les moyens d'assemblage réciproques (60) sont formés, pour partie, sur l'organe de préhension (32) et, pour partie complémentaire, sur l'organe de fixation (44) de sorte à assurer une liaison mécanique structurelle directe entre ledit organe de préhension (32) et ledit organe de fixation (44).

9. Conteneur (10) selon l'une quelconque des revendications 1 à 8, dans lequel les moyens d'assemblage réciproques (160) comprennent un organe intermédiaire de liaison (170) distinct dudit organe de préhension (132) et dudit organe de fixation (144).

10. Conteneur (10) selon la revendication 9, dans lequel l'organe intermédiaire de liaison (170) présente des moyens de solidarisation (172) à l'organe de préhension (132) et/ou dans lequel l'organe intermédiaire de liaison (170) présente une portion formant berceau de réception (175) apte à épouser la forme de la portion d'accouplement (136) de l'au moins un conduit de transfert (22) lorsque ce dernier est enserré par l'organe de préhension (132).

11. Conteneur (10) selon l'une quelconque des revendications 1 à 10, dans lequel le au moins un élément de guidage (30 ; 130) est fixé sur la face interne (12_{INT}) ou la face externe (12_{ExT}) de la paroi périphérique (12) et/ou comprenant n conduits de transfert (22), n étant un entier supérieur à 1, et au moins une entretoise de cloisonnement (198) présentant m sièges de réception (200), m étant un entier supérieur à 1, aptes à recevoir les n conduits de transfert (22) et à empêcher le contact desdits n conduits de transfert (22) les uns avec les autres et/ou dans lequel un premier élément de guidage (30 ; 130) est supporté par une première zone de liaison (46 ; 146) de la paroi périphérique (12) et enserre la portion extrême proximale (22_{PROX}) d'un premier conduit de transfert (22) de sorte à la guider jusqu'à un premier emplacement correspondant à la zone d'introduction ou de retrait d'un premier produit de transfert (20).

12. Procédé de réalisation d'un conteneur (10) pour la réception d'un contenu biopharmaceutique (C) sous forme liquide, fluide ou bien gazeuse, selon l'une quelconque des revendications 1 à 11, **caractérisé en ce qu'**il comprend une succession d'étapes consistant à :
- disposer d'une paroi périphérique (12) destinée à être refermée sur elle-même pour définir une chambre interne de réception (14) capable de recevoir le contenu biopharmaceutique (C) ;
- solidariser structurellement au moins un conduit de transfert (22) présentant une portion extrême proximale (22_{PROX}) et une portion extrême distale (22_{DIST}) à un organe de préhension (32 ; 132) ;
- fixer un organe de fixation (44 ; 144) structurellement distincts et indépendants de l'organe de préhension (32 ; 132) sur n'importe quelle zone de liaison (46 ; 146) prédéterminée de la paroi périphérique (12) sans affecter son étanchéité ;
- puis, assembler de façon structurelle ou fonctionnelle ledit organe de préhension (32 ; 132) avec ledit organe de fixation (44 ; 144) pour former un élément de guidage (30 ; 130) permettant de garantir le positionnement dudit organe de préhension (32 ; 132) et de l'au moins un conduit de transfert (22) qui lui est solidaire à proximité dudit organe de fixation (44 ; 144) et de la zone de liaison (46 ; 146) de la paroi périphérique (12) correspondante.

13. Procédé de réalisation selon la revendication 12, dans lequel :
- soit l'organe de fixation (44 ; 144) présente une base de fixation (48 ; 148) dotée d'une face plate (50 ; 150) qui est fixée sur l'une des zones de liaison (46 ; 146) de la paroi périphérique (12) par un procédé de fixation irréversible tel que le soudage, le collage, ou analogue ;
- soit l'organe de fixation (44 ; 144) présente une base de fixation (148) et une contre-pièce de fixation (186) qui sont positionnées de part et d'autre de la paroi périphérique (12) et sont assemblées mécaniquement l'un à l'autre, de manière réversible ou irréversible, sans traverser la paroi périphérique (12) ni altérer la continuité structurelle de cette dernière de sorte à enserrer l'une des zones de liaison (46 ; 146) de la paroi périphérique (12) et assurer le maintien en position de l'organe de fixation sur ladite zone de liaison (46 ; 146) ;
- soit la paroi périphérique (12) présente au moins une ouverture de fixation (12a) et l'organe de fixation (144) présente une base de fixation (148) et une contre-pièce de fixation (186), l'une ou l'autre de la base de fixation (148) et de la contre-pièce de fixation (186) étant fixée autour de l'ouverture de fixation (12a) de manière à assurer l'étanchéité de la chambre interne de réception (14) définit par la paroi périphérique (12), l'une ou l'autre de ladite base de fixation (148) et de ladite contre-pièce de fixation (186) étant positionnée de manière à traverser l'ouverture de fixation (12a), la base de fixation (148) et la contre-pièce de fixation (186) étant assemblées mécaniquement l'un à l'autre, de manière réversible ou irréversible, de sorte à enserrer l'une des zones de liaison (146) de la paroi périphérique (12) et assurer le maintien en position de l'organe de fixation (144) sur ladite zone de liaison (146).

14. Procédé de réalisation selon l'une des revendications 12 à 13, dans lequel l'organe de préhension (32 ; 132) est solidarisé structurellement à l'au moins un conduit de transfert (22) par le serrage de moyens d'encerclement (34 ; 134) sur une portion d'accouplement (36 ; 136) dudit au moins un conduit de transfert (22) et/ou l'organe de préhension (32 ; 132) et l'organe de fixation (44 ; 144) sont reliés, l'un à l'autre, par une liaison mécanique directe et/ou préalablement à l'assemblage de l'organe de préhension (132) avec l'organe de fixation (144), un organe intermédiaire de liaison (170) distinct dudit organe de préhension (132) et dudit organe de fixation (144) est solidarisé à l'organe de préhension (132) ; cet organe intermédiaire de liaison (170) étant ensuite solidarisé à l'organe de fixation (144) pour garantir le positionnement dudit organe de préhension (132) et de l'au moins un conduit de transfert (22) qui lui est solidaire à proximité dudit organe de fixation (144) et de la zone de liaison (146) de la paroi périphérique (12) correspondante et/ou dans lequel, avant de solidariser structurellement n conduits de transfert (22), n étant un entier supérieur à 1, à l'organe de préhension (32 ; 132), lesdits n conduits de transfert (22) sont placés dans des sièges de réception (200) formés sur une entretoise de cloisonnement (198) de manière à empêcher le contact desdits n conduits de transfert (22) les uns avec les autres et à éviter que l'un de ces n conduits de transfert (22) ne s'écrase sous l'effet de la contrainte de serrage.

15. Procédé de réalisation selon l'une quelconque des revendications 12 à 14, dans lequel :
- une première série d'organes de préhension (32 ; 132) est solidarisée à une première série de portions d'accouplement (36 ; 136) disposées le long d'une première série de conduit(s) de transfert (22) ;
- une première série d'organes de fixation (44; 144), structurellement distincts et indépendants des organes de préhension (32 ; 132), est fixée sur une première série de zones de liaison (46 ; 146) de la paroi périphérique (12) sans affecter son étanchéité et,
- puis chaque organe de préhension (32 ; 132) de la première série est assemblé de façon structurelle ou fonctionnelle sur un organe de fixation (44 ; 144) de la première série afin de guider la première série de conduit(s) de transfert (22) à proximité de la paroi périphérique (12) et jusqu'à un premier emplacement correspondant à la zone d'introduction ou de retrait d'un premier produit de transfert (20).

16. Procédé de réalisation selon la revendication 15, dans lequel :
- soit :
- les organes de préhension (32 ; 132) de la première série sont solidarisés le long de la première série de conduit(s) de transfert (22) de manière à définir, entre les portions d'accouplement (36 ; 136) de la première série, des intervalles de solidarisation prédéterminés,
- les organes de fixation (44 ; 144) de la première série sont fixés sur la paroi périphérique (12) de manière à définir, entre les zones de liaison (46 ; 146) de la première série, des intervalles de liaison prédéterminés et similaires aux intervalles de solidarisation,
- de sorte que, après l'assemblage structurel ou fonctionnel des organes de préhension (32 ; 132) et des organes de fixation (44 ; 144) de la première série, la première série de conduit(s) de transfert (22) définissent une première trajectoire coïncidant avec la trajectoire définie par la première série de zones de liaison (46 ; 146) sur la paroi périphérique (12) ;
- soit :
- les organes de préhension (32 ; 132) de la première série sont solidarisés le long de la première série de conduit(s) de transfert (22) de manière à définir, entre les portions d'accouplement (36; 136) de la première série, des intervalles de solidarisation prédéterminés,
- les organes de fixation (44 ; 144) de la première série sont fixés sur la paroi périphérique (12) de manière à définir, entre les zones de liaison (46 ; 146) de la première série, des intervalles de liaison prédéterminés et inférieurs aux intervalles de solidarisation,
- de sorte que, après l'assemblage structurel ou fonctionnel des organes de préhension et des organes de fixation de la première série, la première série de conduit(s) de transfert définissent une première trajectoire distincte de la trajectoire définie par la première série de zones de liaison (46 ; 146) sur la paroi périphérique (12).

## Patentansprüche

1. Behälter (10) für die Aufnahme eines biopharmazeutischen Inhalts (C) in flüssiger, fluider oder auch gasförmiger Form, umfassend:
eine Umfangswand (12), die in sich selbst geschlossen ist und eine innere Aufnahmekammer (14) definiert, die geeignet ist, den biopharmazeutischen Inhalt (C) aufzunehmen;
mindestens eine Transferleitung (22), die einen offenen proximalen Endabschnitt (22_{PROX}) auf der inneren Aufnahmekammer (14) und einen offenen distalen Endabschnitt (22_{DIST}) am Äußeren des Behälters (10) aufweist, um den Transport eines Transferproduktes (20) zwischen der inneren Aufnahmekammer (14) und dem Äußeren des Behälters (10) zu gestatten;
mindestens ein Führungselement (30; 130), das geeignet ist, von der Umfangswand (12) getragen zu werden und die mindestens eine Transferleitung (22) in Position in der Nähe dieser Umfangswand (12) zu halten, um eine vorbestimmte Bahn zwischen dem proximalen Endabschnitt (22_{PROX}) und dem distalen Endabschnitt (22_{DIST}) vorzugeben;
bei dem das mindestens eine Führungselement (30; 130) einerseits ein Greifelement (32; 132), das geeignet ist, strukturell mit der mindestens einen Transferleitung (22) verbunden zu werden, und andererseits ein Befestigungselement (44; 144) umfasst, das geeignet ist, auf jeder beliebigen vorbestimmten Verbindungszone (46; 146) der Umfangswand (12) befestigt zu werden, ohne ihre Dichtigkeit zu beeinträchtigen;
**dadurch gekennzeichnet, dass** das Greifelement (32; 132) und das Befestigungselement (44; 144) strukturell getrennt und voneinander unabhängig sind, und dass das Führungselement (30; 130) Mittel zum wechselseitigen Zusammenbau (60; 160) aufweist, die es ermöglichen, den strukturellen oder funktionellen Zusammenbau des Greifelements (32; 132), wenn dieses letztgenannte mit der mindestens einen Transferleitung (22) verbunden ist, mit dem Befestigungselement (44; 144) zu gewährleisten, wenn dieses letztgenannte auf der Verbindungszone (46; 146) befestigt ist, um die Positionierung des Greifelements (32; 132) in der Nähe des Befestigungselements (44; 144) und der Verbindungszone (46; 146) der entsprechenden Umfangswand (12) zu gewährleisten.

2. Behälter (10) nach Anspruch 1, bei dem das Befestigungselement (44; 144) eine Befestigungsbasis (48; 148) aufweist, die mit einem ausreichenden inneren Halt versehen ist, um jede strukturelle Verformung bei Nichtvorhandensein einer äußeren Belastung zu vermeiden.

3. Behälter (10) nach Anspruch 2, bei dem die Befestigungsbasis (48; 148) eine flache Seite (50; 150) aufweist, die geeignet ist, auf einer der Verbindungszonen (46; 146) der Umfangswand (12) durch ein Verfahren zur irreversiblen Befestigung, wie Schweißen, Kleben oder dergleichen, befestigt zu werden.

4. Behälter (10) nach Anspruch 2, bei dem die Befestigungsbasis (48; 148) und ein Gegenbefestigungsstück (186) geeignet sind, beiderseits der Umfangswand (12) derart positioniert zu werden, dass sie eine der Verbindungszonen (46; 146) der Umfangswand (12) umspannen und den Halt des Befestigungselements (44; 144) in Position auf der Verbindungszone (46; 146) gewährleisten.

5. Behälter (10) nach Anspruch 4, bei dem:
- entweder die Befestigungsbasis (48; 148) und das Gegenbefestigungsstück (186) komplementäre geometrische Profile aufweisen, die mechanisch miteinander auf reversible oder irreversible Weise verbunden werden können, ohne die Umfangswand (12) zu queren oder die strukturelle Kontinuität dieser letztgenannten zu zerstören;
- oder die Umfangswand (12) mindestens eine Befestigungsöffnung (12a) aufweist, wobei die Befestigungsbasis (48; 148) oder das Gegenbefestigungsstücks (186) geeignet ist, um die Befestigungsöffnung (12a) befestigt zu werden, um die Dichtigkeit der inneren Aufnahmekammer (14), die durch die Umfangswand (12) definiert ist, zu gewährleisten, wobei die (das) andere der Befestigungsbasis (48; 148) und des Gegenbefestigungsstücks (186) geeignet ist, durch die Befestigungsöffnung (12a) hindurchzugehen, wobei die Befestigungsbasis (48; 148) und das Gegenbefestigungsstück (186) komplementäre geometrische Profile aufweisen, die mechanisch miteinander auf reversible oder irreversible Weise verbunden werden können.

6. Behälter (10) nach einem der Ansprüche 1 bis 5, bei dem das Greifelement (32; 132) von Umspannungsmitteln (34; 134) gebildet ist, die geeignet sind, einen Kupplungsabschnitt (36; 136) der mindestens einen Transferleitung (22) zu umspannen.

7. Behälter (10) nach Anspruch 6, bei dem die Umspannungsmittel (34; 134) Spannmittel (40; 140) aufweisen, die geeignet sind, die mindestens eine Transferleitung (22) zu umspannen, wobei sie sich an ihre Dimensionen anpassen, sowie Feststellmittel (42; 142) in der gespannten Position, die geeignet sind, die Spannmittel (40; 140) festzustellen, wenn die Umspannungsmittel (34; 134) an die Dimensionen der mindestens einen Transferleitung (22) angepasst sind, ohne sie unter der Wirkung der Spannkraft zusammenzudrücken oder zu falzen, und/oder bei dem die Umspannungsmittel (34; 134) eine Elastizität aufweisen, die es ihnen ermöglicht, die mindestens eine Transferleitung (22) zu umspannen, ohne sie unter der Wirkung der Spannkraft zusammenzudrücken oder zu falzen.

8. Behälter (10) nach einem der Ansprüche 1 bis 7, bei dem die Mittel zum wechselseitigen Zusammenbau (60) zum Teil auf dem Greifelement (32) und zum komplementären Teil auf dem Befestigungselement (44) ausgebildet sind, um eine direkte strukturelle mechanische Verbindung zwischen dem Greifelement (32) und dem Befestigungselement (44) zu gewährleisten.

9. Behälter (10) nach einem der Ansprüche 1 bis 8, bei dem die Mittel zum wechselseitigen Zusammenbau (160) ein Zwischenverbindungselement (170) umfassen, das von dem Greifelement (132) und dem Befestigungselement (144) getrennt ist.

10. Behälter (10) nach Anspruch 9, bei dem das Zwischenverbindungselement (170) Mittel zur Verbindung (172) mit dem Greifelement (132) aufweist, und/oder bei dem das Zwischenverbindungselement (170) einen Abschnitt aufweist, der eine Aufnahmelagerung (175) bildet, die geeignet ist, sich an die Form des Kupplungsabschnitts (136) der mindestens einen Transferleitung (22) anzulegen, wenn diese letztgenannte von dem Greifelement (132) umspannt wird.

11. Behälter (10) nach einem der Ansprüche 1 bis 10, bei dem mindestens ein Führungselement (30; 130) auf der Innenseite (12_{INT}) oder der Außenseite (12_{EXT}) der Umfangswand (12) befestigt ist, und/oder umfassend n Transferleitungen (22), wobei n eine ganze Zahl größer als 1 ist, und mindestens einen Trennbalken (198), der m Aufnahmesitze (200), wobei m eine ganze Zahl größer als 1 ist, aufweist, die geeignet sind, die n Transferleitungen (22) aufzunehmen und den Kontakt der n Transferleitungen (22) miteinander zu verhindern, und/oder bei dem ein erstes Führungselement (30; 130) von einer ersten Verbindungszone (46; 146) der Umfangswand (12) getragen wird und den proximalen Endabschnitt (22_{PROX}) einer ersten Transferleitung (22) umspannt, um ihn zu einer ersten Stelle entsprechend der Einführungs- oder Entnahmezone eines ersten Transferproduktes (20) zu führen.

12. Verfahren zur Herstellung eines Behälters (10) für die Aufnahme eines biopharmazeutischen Inhalts (C) in flüssiger, fluider oder auch gasförmiger Form nach einem der Ansprüche 1 bis 11, **dadurch gekennzeichnet, dass** es eine Folge von Schritten umfasst, darin bestehend:
- eine Umfangswand (12), die dazu bestimmt ist, in sich selbst geschlossen zu sein, um eine innere Aufnahmekammer (14) zu definieren, die geeignet ist, den biopharmazeutischen Inhalt (C) aufzunehmen, anzuordnen;
- strukturell mindestens eine Transferleitung (22), die einen proximalen Endabschnitt (22PROX) und einen distalen Endabschnitt (22DIST) aufweist, mit einem Greifelement (32; 132) zu verbinden;
- ein Befestigungselement (44; 144), das strukturell getrennt und unabhängig von dem Greifelement (32; 132) ist, auf jeder beliebigen vorbestimmten Verbindungszone (46; 146) der Umfangswand (12) zu befestigen, ohne ihre Dichtigkeit zu beeinträchtigen;
- dann auf strukturelle oder funktionelle Weise das Greifelement (32; 132) mit dem Befestigungselement (44; 144) zusammenzubauen, um ein Führungselement (30; 130) zu bilden, das es ermöglicht, die Positionierung des Greifelements (32; 132) und der mindestens einen Transferleitung (22), die mit ihm verbunden ist, in der Nähe des Befestigungselements (44; 144) und der Verbindungszone (46; 146) der entsprechenden Umfangswand (12) zu gewährleisten.

13. Herstellungsverfahren nach Anspruch 12, bei dem:
- entweder das Befestigungselement (44; 144) eine Befestigungsbasis (48; 148) aufweist, die mit einer flachen Seite (50; 150) versehen ist, die auf einer der Verbindungszonen (46; 146) der Umfangswand (12) durch ein Verfahren zur irreversiblen Befestigung, wie Schweißen, Kleben oder dergleichen, befestigt wird;
- oder das Befestigungselement (44; 144) eine Befestigungsbasis (148) und ein Gegenbefestigungsstück (186) aufweist, die beiderseits der Umfangswand (12) positioniert und mechanisch miteinander auf reversible oder irreversible Weise verbunden sind, ohne die Umfangswand (12) zu queren oder die strukturelle Kontinuität dieser letztgenannten zu zerstören, um eine der Verbindungszonen (46; 146) der Umfangswand (12) zu umspannen und den Halt des Befestigungselements in Position auf der Verbindungszone (46; 146) zu gewährleisten;
- oder die Umfangswand (12) mindestens eine Befestigungsöffnung (12a) aufweist, und das Befestigungselement (144) eine Befestigungsbasis (148) und ein Gegenbefestigungsstück (186) aufweist, wobei die eine oder das andere der Befestigungsbasis (148) und des Gegenbefestigungsstücks (186) um die Befestigungsöffnung (12a) befestigt ist, um die Dichtigkeit der inneren Aufnahmekammer (14), die durch die Umfangswand (12) definiert ist, zu gewährleisten, wobei die eine oder das andere der Befestigungsbasis (148) und des Gegenbefestigungsstücks (186) positioniert ist, um durch die Befestigungsöffnung (12a) hindurchzugehen, wobei die Befestigungsbasis (148) und das Gegenbefestigungsstück (186) mechanisch miteinander auf reversible oder irreversible Weise verbunden sind, um eine der Verbindungszonen (146) der Umfangswand (12) zu umspannen und den Halt des Befestigungselements (144) in Position auf der Verbindungszone (146) zu gewährleisten.

14. Herstellungsverfahren nach einem der Ansprüche 12 bis 13, bei dem das Greifelement (32; 132) strukturell mit der mindestens einen Transferleitung (22) durch das Spannen von Umspannungsmitteln (34; 134) auf einen Kupplungsabschnitt (36; 136) der mindestens einen Transferleitung (22) verbunden wird, und/oder das Greifelement (32; 132) und das Befestigungselement (44; 144) miteinander durch eine direkte mechanische Verbindung verbunden werden, und/oder vor dem Zusammenbau des Greifelements (132) mit dem Befestigungselement (144) ein Zwischenverbindungselement (170), das von dem Greifelement (132) und dem Befestigungselement (144) getrennt ist, mit dem Greifelement (132) verbunden wird; wobei dieses Zwischenverbindungselement (170) sodann mit dem Befestigungselement (144) verbunden wird, um die Positionierung des Greifelements (132) und der mindestens einen Transferleitung (22), die mit ihm verbunden ist, in der Nähe des Befestigungselements (144) und der Verbindungszone (146) der entsprechenden Umfangswand (12) zu gewährleisten, und/oder bei dem vor der strukturellen Verbindung von n Transferleitungen (22), wobei n eine ganze Zahl größer als 1 ist, mit dem Greifelement (32; 132) die n Transferleitungen (22) in Aufnahmesitzen (200), die auf einem Trennbalken (198) ausgebildet sind, angeordnet werden, um den Kontakt der n Transferleitungen (22) miteinander zu verhindern, und zu vermeiden, dass eine dieser n Transferleitungen (22) unter der Wirkung der Spannkraft zusammengedrückt wird.

15. Herstellungsverfahren nach einem der Ansprüche 12 bis 14, bei dem:
- eine erste Reihe von Greifelementen (32; 132) mit einer ersten Reihe von Kupplungsabschnitten (36; 136), die entlang einer ersten Reihe von Transferleitungen (22) angeordnet sind, verbunden wird;
- eine erste Reihe von Befestigungselementen (44, 144), die strukturell getrennt und von den Greifelementen (32; 132) unabhängig sind, auf einer ersten Reihe von Verbindungszonen (46; 146) der Umfangswand (12) befestigt wird, ohne ihre Dichtigkeit zu beeinträchtigen; und
- sodann jedes Greifelement (32; 132) der ersten Reihe strukturell oder funktionell auf einem Befestigungselement (44; 144) der ersten Reihe befestigt wird, um die erste Reihe von Transferleitungen (22) in der Nähe der Umfangswand (12) und bis zu einer ersten Stelle entsprechend der Einführungs- oder Entnahmezone eines ersten Transferproduktes (20) zu führen.

16. Herstellungsverfahren nach Anspruch 15, bei dem:
- entweder:
- die Greifelemente (32; 132) der ersten Reihe entlang der ersten Reihe von Transferleitungen (22) verbunden sind, um zwischen den Kupplungsabschnitten (36; 136) der ersten Reihe vorbestimmte Befestigungsintervalle zu definieren;
- die Befestigungselemente (44; 144) der ersten Reihe an der Umfangswand (12) befestigt sind, um zwischen den Verbindungszonen (46; 146) der ersten Reihe vorbestimmte Verbindungsintervalle ähnlich den Befestigungsintervallen zu definieren;
- so dass nach dem strukturellen oder funktionellen Zusammenbau der Greifelemente (32; 132) und der Befestigungselemente (44; 144) der ersten Reihe die erste Reihe von Transferleitungen (22) eine erste Bahn definiert, die mit der von der ersten Reihe von Verbindungszonen (46; 146) auf der Umfangswand (12) definierten Bahn zusammenfällt;
- oder
- die Greifelemente (32; 132) der ersten Reihe entlang der ersten Reihe von Transferleitungen (22) verbunden sind, um zwischen den Kupplungsabschnitten (36; 136) der ersten Reihe vorbestimmte Befestigungsintervalle zu definieren;
- die Befestigungselemente (44; 144) der ersten Reihe an der Umfangswand (12) befestigt sind, um zwischen den Verbindungszonen (46; 146) der ersten Reihe vorbestimmte Verbindungsintervalle unterhalb der Befestigungsintervalle zu definieren;
- so dass nach dem strukturellen oder funktionellen Zusammenbau der Greifelemente und der Befestigungselemente der ersten Reihe die erste Reihe von Transferleitungen eine erste Bahn definiert, die sich von der von der ersten Reihe von Verbindungszonen (46; 146) auf der Umfangswand (12) definierten Bahn unterscheidet.

## Claims

1. Container (10) for receiving biopharmaceutical content (C) in liquid, fluid or gaseous form, comprising:
a peripheral wall (12), completely closed and defining an inner receiving chamber (14) capable of receiving the biopharmaceutical content (C);
at least one transfer pipe (22) having a proximal end portion (22_{PROX}) opening into the inner receiving chamber (14) and a distal end portion (22_{DIST}) open to the outside of the container (10) to enable a transfer product (20) to be transferred between said inner receiving chamber (14) and the outside of said container (10);
at least one guide element (30; 130) which can be supported by the peripheral wall (12) and hold the at least one transfer pipe (22) in position close to this peripheral wall (12) so as to impose a predetermined path between the proximal end portion (22_{PROX}) and the distal end portion (22_{DIST});
the at least one guide element (30; 130) comprising, on the one hand, a gripping member (32; 132) which can be structurally secured to said at least one transfer pipe (22) and, on the other hand, an attachment member (44; 144) which can be attached to any predetermined connection area (46; 146) of the peripheral wall (12) without compromising its fluid-tightness;
**characterised in that** the gripping member (32; 132) and the attachment member (44; 144) are structurally separate and independent of one another and the guide element (30; 130) has reciprocal assembly means (60; 160) enabling the structural or functional assembly of the gripping member (32; 132) when the latter is secured to the at least one transfer pipe (22) with the attachment member (44; 144) when the latter is attached to the connection area (46; 146) in order to guarantee the positioning of said gripping member (32; 132) close to said attachment member (44; 144) and corresponding connection area (46; 146) of the peripheral wall (12).

2. Container (10) as claimed in claim 1, wherein the attachment member (44; 144) has a mounting base (48; 148) having sufficient intrinsic strength to prevent any structural deformation in the absence of external stress.

3. Container (10) as claimed in claim 2, wherein the mounting base (48; 148) has a flat face (50; 150) which can be attached to one of the connection areas (46; 146) of the peripheral wall (12) by an irreversible attachment method such as welding, gluing or a similar method.

4. Container (10) as claimed in claim 2, wherein the mounting base (48; 148) and a reciprocal assembly means (186) are configured so that they can be positioned on either side of the peripheral wall (12) so as to grip one of the connection areas (46; 146) of the peripheral wall (12) and ensure that the attachment member (44; 144) is held in position on the connection area (46; 146).

5. Container (10) as claimed in claim 4, wherein:
- either the mounting base (48; 148) and the reciprocal assembly means (186) have complementary geometric profiles which can be mechanically assembled with one another, reversibly or irreversibly, without passing through the peripheral wall (12) or altering the structural continuity of the latter;
- or the peripheral wall (12) has at least one attachment opening (12a), one of the mounting base (48; 148) and the reciprocal assembly means (186) being designed to be secured around the attachment opening (12a) in such a way as to ensure the fluid-tightness of the inner receiving chamber (14) defined by the peripheral wall (12), the other of said mounting base (48; 148) and said reciprocal assembly means (186) being designed to extend through the attachment opening (12a), the mounting base (48 ; 148) and reciprocal assembly means (186) having complementary geometric profiles which can be mechanically assembled with one another, reversibly or irreversibly.

6. Container (10) as claimed in any one of claims 1 to 5, wherein the gripping member (32; 132) is formed by encircling means (34; 134) configured to grip a coupling portion (36; 136) of the at least one transfer pipe (22).

7. Container (10) as claimed in claim 6, wherein the encircling means (34; 134) have clamping means (40; 140) configured to clamp the at least one transfer pipe (22) by adjusting to its dimensions and locking means (42; 142) capable of locking the clamping means (40; 140) in the clamped position when the encircling means (34; 134) are adjusted to the dimensions of the least one transfer pipe (22) without crushing or bending it under the effect of the clamping force and/or wherein the encircling means (34; 134) have an elasticity enabling them to clamp the at least one transfer pipe (22) without crushing it or bending it under the effect of the clamping force.

8. Container (10) as claimed in any one of claims 1 to 7, wherein a part of the reciprocal assembly means (60) is disposed on the gripping member (32) and the complementary part is disposed on the attachment member (44) so as to afford a direct structural mechanical connection between said gripping member (32) and said attachment member (44).

9. Container (10) as claimed in any one of claims 1 to 8, wherein the reciprocal assembly means (160) comprise an intermediate connection member (170) separate from said gripping member (132) and said attachment member (144).

10. Container (10) as claimed in claim 9, wherein the intermediate connection member (170) has means (172) for securing to the gripping member (132) and/or wherein the intermediate connection member (170) has a portion forming a receiving cradle (175) configured to match the shape of the coupling portion (136) of the at least one transfer pipe (22) when the latter is clamped by the gripping member (132).

11. Container (10) as claimed in any one of claims 1 to 10, wherein the at least one guide element (30; 130) is secured to the internal face (12_{INT}) or the external face (12_{EXT}) of the peripheral wall (12) and/or comprises n transfer pipes (22), n being a whole number greater than 1, and at least one spacer plate (198) having m receiving seats (200), m being a whole number greater than 1, configured to receive the n transfer pipes (22) and prevent the n transfer pipes (22) from coming into contact with one another and/or wherein a first guide element (30; 130) is supported by a first connection area (46; 146) of the peripheral wall (12) and clamps the proximal end portion (22_{PROX}) of a first transfer pipe (22) so as to guide it to a first location corresponding to the region where a first transfer product (20) is introduced or removed.

12. Method for producing a container (10) for receiving biopharmaceutical content (C) in liquid, fluid or gaseous form, as claimed in any one of claims 1 to 11, **characterised in that** it comprises a succession of steps involving:
- providing a peripheral wall (12) designed to be completely closed in order to define an inner receiving chamber (14) capable of receiving the biopharmaceutical content (C);
- structurally securing at least one transfer pipe (22) having a proximal end portion (22_{PROX}) and a distal end portion (22_{DIST}) to a gripping member (32 ; 132);
- securing an attachment member (44; 144) that is structurally separate and independent of the gripping member (32; 132) to any predetermined connection area (46; 146) of the peripheral wall (12) without compromising its fluid-tightness;
- then, structurally or functionally assembling said gripping member (32; 132) with said attachment member (44; 144) to form a guide element (30; 130) that will guarantee the positioning of said gripping member (32; 132) and the at least one transfer pipe (22) secured thereto close to said attachment member (44; 144) and corresponding connection area (46; 146) of the peripheral wall (12).

13. Production method as claimed in claim 12, wherein:
- either the attachment member (44; 144) has a mounting base (48; 148) having a flat face (50; 150) which is attached to one of the connection areas (46; 146) of the peripheral wall (12) by an irreversible attachment method such as welding, gluing or a similar method;
- or the attachment member (44; 144) has a mounting base (148) and a reciprocal assembly means (186) which are positioned on either side of the peripheral wall (12) and are mechanically assembled with one another, reversibly or irreversibly, without passing through the peripheral wall (12) or altering the structural continuity of the latter so as to grip one of the connection areas (46; 146) of the peripheral wall (12) and ensure that the attachment member is held in position on said connection area (46 ; 146);
- or the peripheral wall (12) has at least one attachment opening (12a) and the attachment member (144) has a mounting base (148) and a reciprocal assembly means (186), one or the other of the mounting base (148) and reciprocal assembly means (186) being secured around the attachment opening (12a) in such a way as to ensure the fluid-tightness of the inner receiving chamber (14) defined by the peripheral wall (12), one or the other of said mounting base (148) and said reciprocal assembly means (186) being positioned so as to extend through the attachment opening (12a), the mounting base (148) and reciprocal assembly means (186) being mechanically assembled with one another, reversibly or irreversibly, so as to grip one of the connection areas (146) of the peripheral wall (12) and ensure that the attachment member (144) is held in position on said connection area (146).

14. Production method as claimed in one of claims 12 to 13, wherein the gripping member (32; 132) is structurally secured to the at least one transfer pipe (22) by the clamping action of the encircling means (34; 134) on a coupling portion (36; 136) of said at least one transfer pipe (22) and/or the gripping member (32; 132) and attachment member (44; 144) are connected to one another by a direct mechanical connection and/or prior to assembling the gripping member (132) with the attachment member (144), an intermediate connection member (170) that is separate from said gripping member (132) and said attachment member (144) is secured to the gripping member (132); this intermediate connection member (170) then being secured to the attachment member (144) in order to guarantee the positioning of said gripping member (132) and the at least one transfer pipe (22) secured thereto close to said attachment member (144) and corresponding connection area (146) of the peripheral wall (12) and/or wherein before structurally securing n transfer pipes (22), n being a whole number greater than 1, to the gripping member (32; 132), said n transfer pipes (22) are placed in receiving seats (200) formed by a spacer plate (198) so as to prevent said n transfer pipes (22) from coming into contact with one another and to prevent one of these n transfer pipes (22) from being crushed under the effect of the clamping force.

15. Production method as claimed in any one of claims 12 to 14, wherein:
- a first set of gripping members (32; 132) is secured to a first set of coupling portions (36; 136) disposed along a first set of transfer pipe(s) (22);
- a first set of attachment members (44; 144), structurally separate and independent of the gripping members (32; 132), is secured to a first set of connection areas (46; 146) of the peripheral wall (12) without compromising its fluid-tightness, and
- then each gripping member (32; 132) of the first set is structurally or functionally assembled with an attachment member (44; 144) of the first set in order to guide the first set of transfer pipe(s) (22) close to the peripheral wall (12) and to a first location corresponding to the region where a first transfer product (20) is introduced or removed.

16. Production method as claimed in claim 15, wherein:
- either:
- the gripping members (32; 132) of the first set are secured along the first set of transfer pipe(s) (22) so as to define predetermined securing intervals between the coupling portions (36; 136) of the first set,
- the attachment members (44; 144) of the first set are secured on the peripheral wall (12) so as to define predetermined connection intervals, similar to the securing intervals, between the connection areas (46; 146) of the first set,
- such that, after structurally or functionally assembling the gripping members (32; 132) and the attachment members (44; 144) of the first set, the first set of transfer pipe(s) (22) defines a first path coinciding with the path defined by the first set of connection areas (46; 146) on the peripheral wall (12);
- or:
- the gripping members (32; 132) of the first set are secured along the first set of transfer pipe(s) (22) so as to define predetermined securing intervals between the coupling portions (36; 136) of the first set,
- the attachment members (44; 144) of the first set are secured on the peripheral wall (12) so as to define predetermined connection intervals that are smaller than the securing intervals between the connection areas (46; 146) of the first set,
- such that after structurally or functionally assembling the gripping members and attachment members of the first set, the first set of transfer pipe(s) defines a first path separate from the path defined by the first set of connection areas (46; 146) on the peripheral wall (12).
